# EUROPÄISCHE PATENTSCHRIFT

(11) **EP 2 053 980 B1**
(45) Veröffentlichungstag und Bekanntmachung des Hinweises auf die Patenterteilung: **07.09.2016**
(21) Anmeldenummer: 07763947.4
(22) Anmeldetag: 16.07.2007
(51) Int. Cl.: A61B 17/80, A61B 17/82, A61B 17/84, A61B 17/74

(54) **TROCHANTER-ZÜGELPLATTE**
TROCHANTER RETENTION PLATE
PLAQUE DE FIXATION DU TROCHANTER

(30) Priorität: 15.08.2006 CH 13092006
(43) Veröffentlichungstag der Anmeldung: 06.05.2009
(73) Patentinhaber: Durst, Heiko, 93138 Lappersdorf (DE)
(72) Erfinder: LORENZ, Kai-Uwe, 9042 Speicher (CH); DURST, Heiko, 9042 Speicher (CH); KUSTER, Markus, 9010 St. Gallen (CH)
(74) Vertreter: Glück Kritzenberger Patentanwälte PartGmbB
(86) Internationale Anmeldenummer: PCT/CH2007/000344
(87) Internationale Veröffentlichungsnummer: WO 2008/019511

(56) Entgegenhaltungen:
- EP-A- 0 847 730
- DE-A1- 10 226 074
- US-A- 5 702 399
- US-A- 6 066 141
- US-A1- 2004 097 942
- US-A1- 2006 058 795

## Beschreibung

Die vorliegende Erfindung betrifft ein Implantat für die Refixation des osteotomierten oder frakturierten Trochanter major, eine so genannte Trochanter-Zügelplatte, zur operativen Refixation des osteotomierten oder frakturierten Trochanter major.

Seit der breiten Einführung der Hüftendoprothetik in den 50-er und 60-er Jahren wurden verschiedene Zugangswege zum Hüftgelenk, unter anderem mit Osteotomie und anschliessender Refixation des Trochanter major entwickelt. Die Refixation dieses wichtigen Sehnenansatzes für den Musculus gluteus medius und Musculus gluteus minimus sowie verschiedener Aussenrotatoren sollte nach der Prothesenimplantation anatomisch erfolgen. Ausserdem sollte die Refixation dieses wichtigen Sehnenansatzes der Zugkraft der Abduktoren, die unter Umständen das Dreifache des Körpergewichtes betragen können, stand halten. Zu diesem Zweck wurden verschiedene Techniken entwickelt. Dazu zählen beispielsweise die Cerclage-Techniken, welche mit Drähten oder Kabelseilen arbeiten. Diese können durch zusätzliche Implantate ergänzt werden. Diese Konstruktionen erreichen Reposition und Kompression der vorhandenen Fragmente mittels Zugurtung/Cerclage über der Osteotomie/Frakturstelle. Eine eigentliche Neutralisation des Abduktorenzugs erfolgt entweder über eine Art Hosenträger-Cerclage, welche am proximalen Femur fixiert ist, oder gar nicht. Die Implantate dienen der Fixierung der Drähte/Kabel am cranialen-lateralen Aspekt des Trochanter major. Eine direkte, stabile Fixierung der Implantate am proximalen Femur erfolgt nicht.

Eine weitere Technik benutzt plattenförmige Implantate mit Zuggurtungsfunktion, welche am lateralen Aspekt des proximalen Femur mit Schrauben oder Cerclagen fixiert sind. Die Reposition und Fixation des Trochanter major erfolgt meist über zinkenartige Ausläufer der plattenförmigen Implantate, die entweder nur in den Trochanter einhaken oder die über den Trochanter bogenförmig auslaufen. Eine Fixierung im Trochanterbereich erfolgt entweder nur mit Zinken und mit Cerclagen um das Trochantermassiv oder mit Schrauben in das Trochanter-major-Fragment. Die lateral liegenden plattenförmigen Implantate als ausschliessliche Kraftaufnehmer erfordern ein stabiles, teilweise hochprofiles Design, um den Abduktorenkräften standzuhalten. Die laterale Lage direkt über dem Tuberculum innominatum kann dann zur Irritation des Tractus iliotibialis führen, der über diesen Bereich gleiten muss. Ferner besteht die Gefahr des Aufbiegens der bogenförmigen Zinken bei grösseren Radien, bei einem Zinkenverlauf um das Trochanter-major-Fragment, oder des Einschneidens der teilweise scharfkantigen Zinken bei osteoporotischen Knochen.

Ein solches Implantat zeigt die US 2004/097942 A1 (Basis für den Oberbegriff des Anspruchs 1). Vorgeschlagen wird ein Implantat mit Kabelklemmen, die ein zerstörungsfreies Klemmen und Lösen eines Kabels möglich machen. Die Klemmverbindung ist so gestaltet, dass das Kabel mit einer Zugkraft beaufschlagt werden kann. Die Kabelklemme ist für den Operateur während des operativen Eingriffs einfach und schnell handhabbar. Das eingeklemmte Kabel kann bei Bedarf während der Operation nachgezogen und so an den individuellen Knochenbau des Patienten angepasst werden. Die Kabelklemme ist so ausgeführt, dass sie während des operativen Vorgangs bei Bedarf wiederverwendet werden kann.

Die EP-A-0 847 730 schlägt ein Implantat mit einer Knochenfixierungsvorrichtung umfassend ein Halteelement aus biokompatiblen Material vor. Das Halteelement weist zwei Seiten auf, wobei an jeder Seite eine Kabelführung vorgesehen ist. Die Kabel sind in einem vordefinierten Winkel am Halteelement fixiert, sodass eine präzise Umschliessung des Knochens möglich gemacht wird. Halteelement und Kabel bilden dabei eine Einheit. Beim Einsatz der Knochenfixierungsvorrichtung umlaufen die Kabel Teile des grossen Trochanter und werden an ihrem freien Ende mittels einer Klemmverbindung verbunden. Zusätzlich sind zur besseren Fixierung am Halteelemente auf jener Seite die dem Knochen zugewandt ist Widerhaken vorgesehen.

Aus der US 6 066 141 A ist eine Trochanter-Zügelplatte für die Refixation des osteotomierten oder frakturierten Trochanter major bekannt, wobei eine am proximalen Femur fixierbare Basisplatte vorgesehen ist, welche kraftschlüssig am Femur gehalten wird und als Basis einer über den Trochanter major verlaufenden Zuggurtungskonstruktion dient, die einen Zügel umfasst und den osteotomierten oder frakturierten Trochanter major form- und/oder kraftschlüssig am Femur refixieren kann.

Eine andere Technik bedient sich Implantaten, die direkt am Prothesenkörper, am Marknagel oder an der dynamischen Hüftschraube (DHS) fixiert sind. Viele der genannten Implantate haben in klinischen und/oder biomechanischen Studien keine ausreichende Stabilität gezeigt, oder viele der genannten Implantate wiesen ein Materialversagen im Verlauf der dynamischen Beanspruchung auf.

Es ist daher die Aufgabe der vorliegenden Erfindung, ein Implantat zur Refixation des osteotomierten oder frakturierten Trochanter major zur Verfügung zu stellen, das die Nachteile der bekannten Implantate vermeidet.

Diese Aufgaben werden gelöst durch eine Trochanter-Zügelplatte gemäss dem unabhängigen Patentanspruch 1.

Trochanter-Zügelplatten gemäss der vorliegenden Erfindung erlauben es die Abduktorenkräfte zu neutralisieren, ohne in die Knochensubstanz des Trochanter major signifikant einzuschneiden. Dies ist insbesondere bei vorliegender Osteoporose von wesentlichem Vorteil.

Der Begriff Platte soll in der Beschreibung und den Ansprüchen zur vorliegenden Erfindung Platten im engeren Sinne, aber auch plattenähnliche Konstruktionen, zum Beispiel verstärkte Geflechte aus verschiedenen, geeigneten Materialien oder mehrteilige miteinander wirkverbundene Platten umfassen.

Geeignete Materialien für Platten, Schrauben, Bolzen, Kabel, Bänder des Implantats, respektive der Trochanter-Zügelplatte, gemäss der vorliegenden Erfindung sind vorzugsweise ausgewählt aus der Gruppe von Edelstahl, Edelstahllegierungen, Titan, Titanlegierungen, medizinischen Kunststoffen, Karbon, Kevlar, Composite-Werkstoffe und bioresorbable Werkstoffe. Letztere haben den Vorteil, dass ein operativer Eingriff zum Entfernen entfällt, weisen aber für viele Anwendungen nur eine ungenügende Stabilität auf. Verschiedene Kombination aus den vorgenannten Materialien sind möglich und vorteilhaft. So kann zum Beispiel durch die Auswahl verschiedener geeigneter Werkstoffe der distale Plattenschaft starr, der mindestens eine proximale Plattenzinken flexibel gestaltet sein.

Die Platte ist gemäss bevorzugter Ausführungsformen massiv gestaltet oder aus einem, gegebenenfalls verstärkten Geflecht gefertigt. Auch kann die Platte kombiniert aus massiven oder geflochtenen Anteilen bestehen, so dass wie bereits oben angeregt, Materialeigenschaften, wie zum Beispiel die Elastizität, lokal modifiziert sein können. Die Porosität des Plattenmaterials kann ebenfalls bereichsweise modifiziert werden, was ebenfalls eine Anpassung lokaler Eigenschaften wie zum Beispiel der Festigkeit und/oder der Elastizität erlaubt.

Nachstehend werden Ausführungsformen der vorliegenden Erfindung anhand der beiliegenden Zeichnungen näher erläutert. Es zeigen:
- Fig. 1: eine Frontansicht der, eine Basisplatte aufweisenden, Trochanter-Zügelplatte, welche am proximalen Femur einer linken Hüfte angebracht ist, wobei die Basisplatte einen unteren Abschnitt aufweist;
- Fig. 2: das Implantat gemäss Fig. 1 in einer Seitenansicht;
- Fig. 3: das Implantat aus Fig. 1 bzw. 2 in einer Ansicht von dorso-medio-cranial;
- Fig. 4: einen vereinfachten Ausschnitt aus dem unteren Abschnitt der Basisplatte aus Fig. 1, welcher mittels Fixiervorrichtungen am Knochen fixiert ist;
- Fig. 5: eine erste Ausführung der Fixiervorrichtung aus Fig. 4 in einer Seitenansicht;
- Fig. 6: eine zweite Ausführung der Fixiervorrichtung in einer Seitenansicht;
- Fig. 7: eine weitere Ausführungsform des erfindungsgemässen Implantats, bei dem der untere Abschnitt der Basisplatte als langer schmaler Plattenschaft ausgebildet ist;
- Fig. 8: eine weitere Ausführungsform des erfindungsgemässen Implantats, bei dem der untere Abschnitt der Basisplatte als gegabelter Plattenschaft mit zwei parallelen voneinander beabstandeten Längsflügeln ausgebildet ist;
- Fig. 9: eine weitere Ausführungsform des erfindungsgemässen Implantats, bei dem der untere Abschnitt der Basisplatte als langer breiter Plattenschaft ausgebildet ist;
- Fig. 10: eine weitere Ausführungsform des erfindungsgemässen Implantats, bei dem die Basisplatte als langer schmaler Plattenschaft mit sechs senkrecht abstehenden Lateralflügeln ausgebildet ist;
- Fig. 11: eine weitere Ausführungsform des erfindungsgemässen Implantats, bei dem ein Quersteg des oberen Abschnitts der Basisplatte nicht ausgebildet ist;
- Fig. 12a bis 12c: eine schematische Darstellungen der Einführung eines Implantats gemäss Figur 11 von distal des Ursprungs des M. vastus lateralis;
- Fig. 13a bis 13c: eine schematische Darstellungen der Einführung eines Implantats gemäss Figur 7 von proximal nach Einschneiden eines kurzen Anteils des Ursprungs des M. vastus lateralis;
- Fig. 14 a: eine weitere Ausführungsform des erfindungsgemässes Implantats, bei dem der Klemmvorrichtung vorgeschaltete Führungsausläufer nicht ausgebildet sind;
- Fig. 14b bis d: das Implantat gemäss Fig. 14a in Ansichten von ventral, dorsal und proximal;
- Fig. 14e und f: das Implantat gemäss Fig. 14a in perspektivischen Ansichten von dorsomedial und ventro-lateral;
- Fig. 15: eine Detailvergrösserung F des distalen Schaftendes des Implantats gemäss Fig. 14a in der Ansicht gemäss Fig. 14e;
- Fig. 16: eine Detailvergrösserung D des proximalen Endbereichs des Implantats gemäss Fig. 14a in der Ansicht gemäss Fig. 14f;
- Fig. 17: eine Detailvergrösserung E des proximalen Bereichs des Implantats gemäss Fig. 14a in der Ansicht gemäss Fig. 14e;
- Fig. 18a und 18b: Implantat gemäss Fig. 14a zusammen mit einer Klemmplatte gemäss einer weiteren Ausführungsform der Erfindung in perspektivischen Explosionsansichten von dorso-medial und dorso-lateral; und
- Fig. 19: ein Implantat gemäss einer weiteren Ausführungsform in Wirkverbindung mit einer Impantationshilfe in Form eines Zielbügels mit einer Mehrzahl von Bohrhülsen verschiedener Durchmesser.

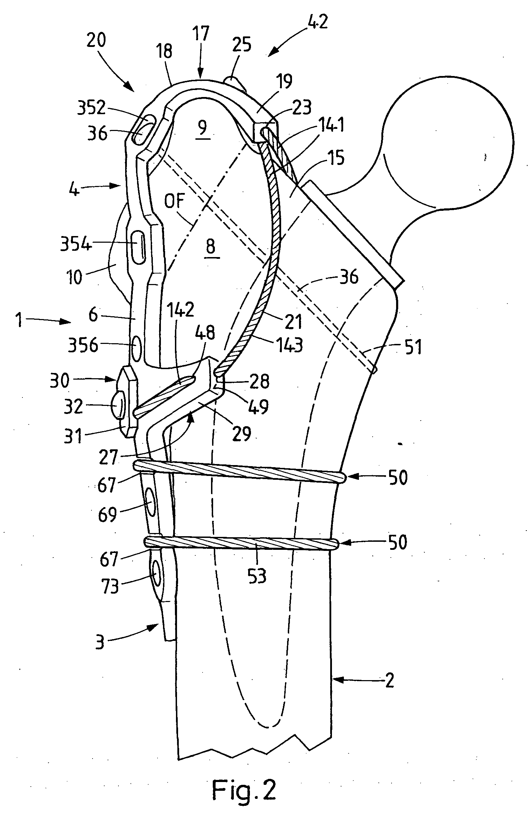

Die Trochanter-Zügelplatte, oben und im Folgenden auch kurz Implantat genannt, dient unter anderem zur anatomischen Reposition und stabilen Fixation des Trochanter major 9 an den proximalen Femur 2 nach Osteotomie oder Fraktur. Eine Osteotomie- bzw. Frakturebene des Trochantermajor-Fragments ist in Fig. 2 mit OF bezeichnet. Die gezeigte Ebene entspricht im Wesentlichen einer Standard-Osteotomie. Bein einer Ganz-Osteotomie würde die Osteotomie-Linie eine Ebene definieren, die bei annähernd gleichem proximalem Ausgangspunkt weiter nach distal geführt wäre. Bei der so genannten "Extended Trochanteric Osteotomy" (ETO) ist die Osteotomie-Linie noch wesentlich weiter nach distal bis in den proximalen Femur geführt.

Die vorliegende Erfindung ist im dargestellten Ausführungsbeispiel basierend auf einem Zuggurtungsprinzip mit zwei Zügeln realisiert, welches aus einer Kombination einer lateral liegenden Platte und medial liegenden Zügeln resultiert.

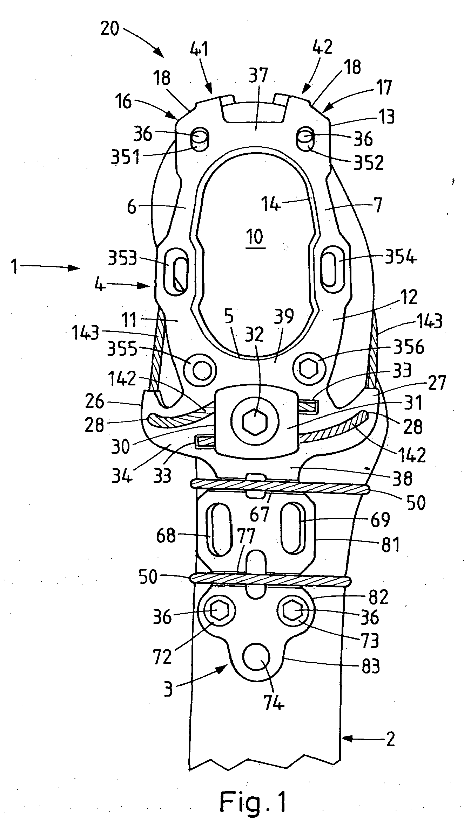

Das Implantat gemäss einer ersten Ausführungsform der Erfindung umfasst eine Basisplatte 1 (Fig. 1 und 2), welche im dargestellten Fall dem lateralen proximalen Femur 2 zugeordnet ist. Die Basisplatte 1 ist im Wesentlichen flach und sie kann somit unter dem Musculus vastus lateralis und um das Tuberculum innominatum 10 gut angeordnet sein. Die Basisplatte 1 umfasst einen unteren, d.h. vom oberen Ende des Schenkelknochens 2 abgewandt liegenden Abschnitt 3 sowie einen oberen, d.h. zum oberen Ende des Schenkelknochens 2 näher liegenden Abschnitt 4.

Der obere Abschnitt 4 der Basisplatte 1 weist einen flächenhaften Hauptteil 5 auf, welcher länglich gewölbt ist. Die Längsachse dieser Wölbung fällt mit der Längsachse der Krümmung der Aussenfläche des Schenkelknochens 2 zusammen oder sie läuft zumindest parallel dazu. Der Verlauf der Wölbung dieses oberen Abschnitts 4 der Basisplatte 1 soll dem Verlauf der Wölbung der Oberfläche jenes Abschnittes des Schenkelknochens 2 entsprechen, welchem dieser Abschnitt 4 der Basisplatte 1 zugeordnet werden soll. Es versteht sich, dass der Radius der Krümmung dieses Abschnitts 4 der Basisplatte 1 entlang ihrer praktisch vertikal verlaufenden Längsachse veränderlich sein kann, je nach dem, wie sich die Krümmung der Oberfläche des Schenkelknochens 2 in seiner Längsrichtung ändert. Die Basisplatte 1 umfasst ferner zwei sich in einem Abstand voneinander befindliche schmale Schenkel 6 und 7, welche sich an den oberen Rand 39 des oberen Abschnittes 4 der Basisplatte 1 einerends anschliessen und von diesem Rand 37 abgehen. Diese Schenkel 6 und 7 sind mit dem oberen Abschnitt 4 der Basisplatte 1 einstückig und sie erstrecken sich vom oberen Abschnitt 4 der Basisplatte 1 aufwärts. Der jeweilige Schenkel 6 bzw. 7 liegt ventral bzw. dorsal vom Tuberculum innominatum 10. Die Schenkel 6 und 7 haben einen im Wesentlichen bogenförmig verlaufenden Grundkörper 11 bzw. 12 und sie sind zueinander konvex angeordnet. Die Basisplatte 1 umfasst auch einen flächenhaften Steg 13, welcher über seinen unteren Rand an die oberen, freien Endpartien der Schenkel 6 und 7 angeschlossen ist. Der Steg 13 überbrückt diese Endpartien der Schenkel 6 und 7 und er verbindet sie miteinander mechanisch. Die beiden Schenkel 6 und 7 sind somit proximal des Tuberculum innominatum 10 mittels des Steges 13 miteinander verbunden.

Die obere Randpartie 39 des Grundkörpers 5 des oberen Plattenabschnitts 4 sowie die Innenkanten der Schenkel 6 und 7 und des Steges 13 begrenzen eine Öffnung 14 in der Basisplatte 1, welche einen praktisch ovalen Umriss hat. In dieser Öffnung 14 kann das Tuberculum innominatum 10 liegen, wenn das Implantat am Femur 2 angebracht ist. Die Schenkel 6 und 7 umgehen somit das Tuberculum innominatum 10, so dass der Tractus iliotibialis problemlos und ungestört gleiten kann.

Der proximale Bereich 4 der Basisplatte 1 weist vier Langlöcher 351, 352, 353 und 354 auf, durch welche Schrauben 36 hindurchgehen, mit deren Hilfe die Basisplatte 1 auf dem Knochen 2 befestigbar ist. Jeweils zwei dieser Langlöcher 351 und 353 bzw. 352 bzw. 354 sind in einem der Schenkel 6 bzw. 7 der Basisplatte 1 ausgeführt, und zwar in einem vertikalen Abstand voneinander. Das erste Langloch 351 bzw. 352 des jeweiligen Lochpaares liegt dort, wo der betreffenden Schenkel 6 bzw. 7 mit dem Steg 13 zusammentrifft. Das zweite Langloch 353 bzw. 354 des jeweiligen Lochpaares befindet sich etwa in der Mitte der Länge des betreffenden Schenkels 6 bzw. 7 der Basisplatte 1. Die Längsachse der Langlöcher 351 bis 354 ist caudal-medial ausgerichtet.

Die Basisplatte 1 ist mit Hilfe von Schrauben 36 am proximalen Femur 8 befestigt, welche durch die Löcher 351 bis 354 hindurchgehen. Diese Schrauben 36 können bloss durch die Oberfläche des Knochens 2 hindurchgehen oder sie können so lang ausgeführt sein, dass sie durch das Trochanter-Fragment 9 hindurchgehen und dass der vordere Bereich 51 des Gewindebolzens der Schraube 36 im proximalen Femur 8 eingeschraubt ist (Fig. 2). Falls eine solche Schraube 36 durch das Trochanter-Fragment 9 hindurchgeht, ohne dass das Gewinde des Schraubbolzens mit dem Material des Trochanter-Fragments 9 in Eingriff steht, so nennt man eine solche Schraube Zugschraube.

Die Ausrichtung der Schraubenlöcher 351 bis 354 in Verbindung mit deren Langlochform ermöglichen eine spitzwinklige, nach caudal medial gerichtete Lage der Schrauben 36, um das Trochanter-Fragment 9 über die Osteotomie oder Frakturebene OF im proximalen Femur 8 zu fixieren. Im Bedarfsfall können diese Schrauben 36 in der Basisplatte 1 winkelstabil eingebracht werden. Dies bedeutet, dass die Unterseite des Kopfes der Schraube 36 konisch ausgeführt ist und dass die Aussenfläche dieses Kopfabschnittes mit einem Gewinde versehen ist. Es ist zweckmässig, die Seitenflächen der Aussparungen 351 bis 354 in der Basisplatte 1 konisch bzw. zusammenlaufend auszuführen, sodass die Aussparungen 351 bis 354 schräg verlaufende Flanken aufweisen. Das Material des Kopfes der Schraube 36 ist härter als das Material der Basisplatte 1, sodass sich das Gewinde am konischen Abschnitt des Schraubenkopfes beim Einschrauben der Schraube 36 in das Material der Basisplatte 1 einschneidet und dadurch eine unveränderliche Winkellage der Schraube 36 gegenüber der Basisplatte 1 sicherstellt. Eine solche Verbindung zwischen der betreffenden Partie der Basisplatte 1 und der Schraube 36 kann wechselnden Belastungen auch über längere Zeiträume Stand halten.

Ist die direkte Verschraubung nicht möglich, kann das Trochanter-Fragment 9 über Schraubelemente an den proximalen Bereich 4 der Basisplatte 1 fixiert werden. Dabei ist die Verwendung der genannten winkelstabilen 36 von Vorteil. Im oberen Randbereich 39 des Hauptteils 5 des proximalen Abschnitts 4 der Basisplatte 1 sind weitere zwei Löcher 355 und 356 ausgeführt. Je eines dieser Löcher 355 und 356 befindet sich dort, wo die Schenkel 6 bzw. 7 mit diesem oberen Randbereich 39 des Hauptteils 5 des proximalen Abschnitts 4 der Basisplatte 1 zusammentrifft. Auch durch diese Löcher 355 und 356 können Schrauben 36 hindurchgeführt und im proximalen Femur 2 eingeschraubt werden.

Das Implantat weist ferner eine Vorrichtung 20 zum kraftschlüssigen Halten des Trochanter major 9 am Femur 2 auf. Diese Haltevorrichtung 20 umfasst im dargestellten Fall zwei im Wesentlichen biegsame Zügel 41 und 42. Der jeweilige Zügel 41 bzw. 42 besitzt einen streifenförmigen Zinken 16 bzw. 17. Diese Zinken 16 und 17 befinden sich in einem Abstand voneinander. Die Zinken 16 und 17 gehen proximal des Steges 13 ab und sie sind so ausgeführt, dass sie sich der Anatomie des Trochanter major anpassen lassen. Zu diesem Zweck ist der gesamte Zinken 16 bzw. 17 oder zumindest ein Bereich 43 des jeweiligen Zinkens 16 bzw. 17 biegsam ausgeführt. Die biegsamen Zinken 16 und 17 weisen einen bogenförmigen Verlauf auf, wobei dieser Bogen aufwärts gerichtet ist. Man kann somit auch sagen, dass der Zinken 16 bzw. 17 in Hakenform vorgebogen ist. Deswegen kann das Trochanter-Fragment 9 von proximal gut umschlossen bzw. ergriffen und unter manuellem Zug nach distal eine gute Reposition des Trochanter-Fragments 9 erreicht werden. Um ein Einschneiden der Zinken 16 und 17 in das Trochanter-major-Fragment möglichst klein zu halten, sollen die Zinken 16 und 17 deutlich breiter als hoch ausgeführt sein. Die Zinken 16 und 17 können auch so ausgeführt sein, dass die Steifheit des jeweiligen Zinkens 16 bzw. 17 mit dem zunehmenden Abstand vom Steg 13 abnimmt, sodass der Zinken 16 bzw. 17 mit dem zunehmenden Abstand vom Steg 13 nachgiebiger wird. Möglich ist auch eine Ausführung der Zinken 16 und 17, bei welcher die Veränderung der-Biegsamkeit derselben dadurch erreicht wird, dass Materialien mit unterschiedlicher Flexibilität für die einzelnen Abschnitte der Zinken 16 und 17 verwendet werden.

Die in den Zeichnungen dargestellten Zinken 16 bzw. 17 weisen eine erste Endpartie 18, eine zweite Endpartie 19 und einen Mittelteil 43, welcher sich zwischen diesen Endpartien 18 und 19 erstreckt. Die erste Endpartie der länglichen Zinkenbasis 18 ist an den Steg 13 angeschlossen und sie ist mit diesem zweckmässigerweise einstückig ausgeführt. Die zweite Endpartie 19 der Zinken 16 und 17 ist als ein verdickter Fortsatz ausgeführt und sie befindet sich medial der Sehnenplatte des Musculus gluteus medius und medial der Spitze des Trochanter-major-Fragments 9.

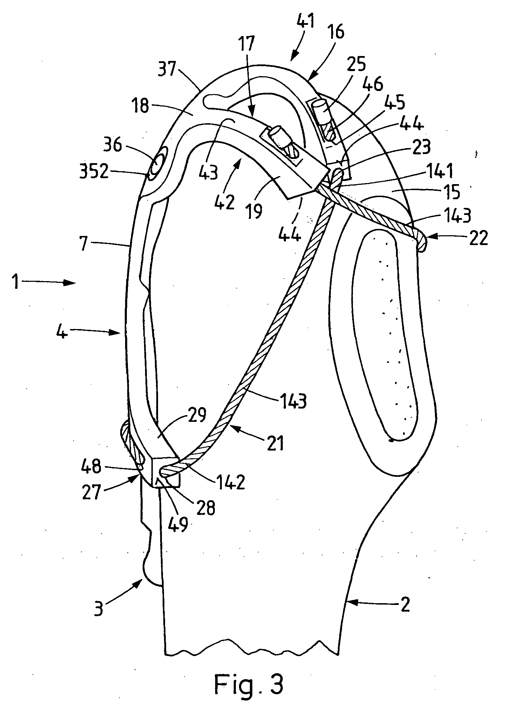

Die Breite des verdickten Fortsatzes 19 gleicht praktisch der Breite des Zinkens 16 bzw. 17. Die Höhe des verdickten Fortsatzes 19 stellt dagegen ein Vielfaches der Dicke der Mittelpartie 43 des Zinkens 16 bzw. 17 dar. Der Fortsatz 19 weist eine Stirnfläche 44 auf (Fig. 2 und 3). Eine Bohrung 23 verläuft durch die Verdickung 19. Eine der Mündungen dieser Bohrung 23 liegt in der Stirnfläche 44 der Verdickung 19. Die Bohrung 23 verläuft durch die Verdickung 19 schräg aufwärts, sodass eine zweite Mündung 45 der Bohrung 23 in der Oberfläche der Verdickung 19 bzw. des Zinkens 16 bzw. 17 liegt.

Der jeweilige Zügel 41 bzw. 42 besitzt ferner ein flexibles, längliches Glied 21 bzw. 22, welches zweckmässigerweise einen kreisrunden Querschnitt aufweist. Der Durchmesser des jeweiligen Längsgliedes 21 bzw. 22 ist kleiner als die Breite des jeweiligen Zinkens 16 bzw. 17. Im dargestellten Fall sind diese Längsglieder 21 und 22 als Seile ausgeführt. Diese Seile 21 und 22 können Kabel sein, und sie können aus Edelstahl, Titan bzw. Kunststoff sein. Die erste Endpartie 141 eines solchen Längsgliedes bzw. Seiles 21 bzw. 22 ist an die zweite, freie Endpartie 19 des entsprechenden Zinkens 16 bzw. 17 angeschlossen (Fig. 1 und 3).

Die zweite bzw. freie Endpartie 142 des jeweiligen Längsgliedes bzw. Seils 21 bzw. 22 ist, nachdem sich die Seile 21 bzw. 22 gekreuzt haben, an der Basisplatte 1 befestigt. Die erste Endpartie 141 des Seils 21 bzw. 22 geht durch die Bohrung 23 in der Verdickung 19 des betreffenden Zinkens 16 bzw. 17 hindurch, wobei ein Endabschnitt 46 dieser ersten Endpartie 141 aus der oberen Mündung 45 der Bohrung 23 im Zinken 16 bzw. 17 herausragt. Dieser von der Bohrungsmündung 45 rückwärts ragende Endabschnitt 46 ist mit einer Kappe 25 versehen, welche an diesem Seilendabschnitt 46 angebracht ist. Die Kappe 25 verhindert, dass dieser Seilendabschnitt 46 durch die Bohrung 23 in der Verdickung 19 des Zinkens 16 bzw. 17 hindurchgeht. Im Bereich der oberen Mündung 45 der Bohrung 23 kann eine Vertiefung in der Oberseite der Verdickung 19 ausgeführt sein, in welcher die Kappe 25 Platz finden kann. Diese Vertiefung in der Zinkenendpartie 19 kann so tief ausgeführt sein, dass die obere Kontur der Kappe 25 unterhalb der Oberfläche dieser Zinkenendpartie 19 liegt. Andere Optionen der Verbindung zwischen diesem Zinkenende 19 und dem Ende 141 des Kabelseils 21 bzw. 22 sind feste Pressverbindungen oder bewegliche Ösen, Gelenkkonstruktionen usw.. Nach dem Austritt aus der Stirnfläche 44 der Zinken 16 und 17 kreuzen sich die Seile 21 und 22 über der Fossa trochanterica 15. Die Folge davon ist, dass jenes Seil 21, welches an dem ventral liegenden Zinken 16 angeschlossen ist, hinsichtlich der Fossa trochanterica 15 nach dorsal und distal verläuft. Jenes Seil 22, welches an dem dorsal liegenden Zinken 17 angeschlossen ist, hinsichtlich der Fossa trochanterica 15 nach ventral und distal verläuft. Die über der Fossa trochanterica 15 gekreuzten Seile 21 und 22 verblocken sich gegeneinander unter Zug an den freien Enden 19 der Zinken 16 bzw. 17. Nach der Überkreuzung werden die Seile 21 und 22 zur Basisplatte 1 geführt, wo ihre zweiten bzw. freien Endpartien 142 durch eine Klemmvorrichtung 30 gehalten sind.

Die Klemmvorrichtung 30 ist an der Aussenseite der Basisplatte 1 und praktisch zwischen dem unteren Abschnitt 3 und dem oberen Abschnitt 4 der Basisplatte 1 angeordnet. Die Klemmvorrichtung 30 (Fig. 1 und 2) umfasst eine Klemmplatte 31 und im dargestellten Fall eine Schraube 32, Vorteilhafterweise eine Feingewindeschraube. Von besonderem Vorteil ist, wenn der Gewindebolzen dieser Feingewindeschraube 32 einen möglichst grossen Durchmesser hat. Die Basisplatte 1 weist in diesem Bereich derselben eine Gewindebohrung (nicht dargestellt) auf, in welcher der Gewindebolzen der Schraube 32 eingeschraubt sein kann. Die zweiten Endpartien 142 der Seile 21 und 22 liegen zwischen der Basisplatte 1 und der Klemmplatte 31. Dabei liegt die zweite Endpartie 142 des ersten Seiles 21 unterhalb des Schraubbolzens der Schraube 32 und die zweite Endpartie 142 des zweiten Seils 22 liegt oberhalb des Schraubbolzens der Schraube 32. Um die Höhe dieses Bereichs des Implantats möglichst niedrig zu halten, sind Vertiefungen 33 in der Basisplatte 1 und/oder der Klemmplatte 31 ausgeführt, wo sich die zweiten Endpartien 142 der Seile 21 und 22 befinden. Diese Vertiefungen 33 nehmen die Endpartien 142 der Seile 21 und 22 auf.

Es hat sich allgemein als vorteilhaft erwiesen je eine längliche Vertiefung oberhalb und unterhalb des Gewindelochs in der Aussenseite des Plattenhauptteils zur Aufnahme der jeweiligen freien Endpartie der Seile anzubringen. Diese Vertiefungen erstrecken sich vorzugsweise beinahe senkrecht zur Längsrichtung der Plattenschenkel und sind nur so tief ausgeführt, dass das in der jeweiligen Vertiefung eingelegte Seil zwischen dem Boden dieser Vertiefung und der Klemmplatte geklemmt sein kann, wenn die Schraube angezogen ist

Wie im Folgenden noch beschrieben wird, greifen gemäss weiterer bevorzugten Ausführungsformen die Zügel, respektive die Längsglieder nicht an den freien Endpartien der entsprechenden Zinken an, sondern an einer in Richtung Zinkenbasis zurückverlagerten Position. In bevorzugten Ausführungsformen greifen sie an der Zinkenbasis selbst an, sind entlang der Zinken geführt, und durchsetzen die an der jeweiligen freien Endpartie der Zinken angeordnete Verdickung. Wird am freien Ende eines Längsglieds gezogen, so wird der jeweilige Zinken unter Einfluss des Längsglieds gebogen, wobei das Längsglied die Kraft gleichmässiger über die Länge in den Zinken einleitet.

Damit die Seile 21 und 22 durch die Befestigung in der Klemmvorrichtung 30 nicht geknickt werden, sind längliche Ausläufer 26 und 27 zur Führung der hier liegenden zweiten Endpartien 142 der Seile 21 und 22 vorgesehen (Fig. 1 bis 3). Der erste Ausläufer 26 ist ventral am Trochantermassiv nach medial gerichtet und der zweite Ausläufer 27 ist dorsal am Trochantermassiv nach medial gerichtet. Diese Führungsausläufer 26 und 27 sind der Klemmvorrichtung 30 vorgeschaltet. Der jeweilige Führungsausläufer 26 bzw. 27 steht von einem der Seitenränder des oberen Abschnittes 4 der Basisplatte 1 ab. Der jeweilige Führungsausläufer 26 bzw. 27 schliesst sich an den Hauptteil 5 der Basisplatte 1 zwischen der oberen horizontal verlaufenden Randpartie 39 dieses Hauptteils 5 und der unteren horizontal verlaufenden Randpartie 38 desselben an.

Der längliche Grundkörper des jeweiligen Führungsausläufers 26 bzw. 27 ist in zwei Richtungen gebogen. Die erste Biegung des länglichen Führungsausläufers 26 bzw. 27 liegt etwa in einer horizontalen Ebene und der Verlauf derselben entspricht etwa dem Verlauf der Biegung der Oberfläche des Knochens 2, dem der Ausläufer 26 bzw. 27 zugeordnet werden soll. Die zweite Biegung des länglichen Führungsausläufers 26 bzw. 27 liegt etwa in einer vertikalen Ebene. Der jeweilige Führungsausläufer 26 bzw. 27 ist in dieser vertikalen Ebene aufwärts gebogen, und zwar entsprechend dem Verlauf der zweiten Endpartien 142 der sich kreuzenden Seile 21 und 22, die in diesem Bereich der Basisplatte 1 durch die Klemmvorrichtung 30 gehalten sind.

Die freiliegende Endpartie 29 des gebogenen Führungsausläufers 26 bzw. 27 ist im dargestellten Fall dicker als jene Endpartie 34 des Führungsausläufers 26 bzw. 27, welche an den Hauptteil 5 der Basisplatte 1 angeschlossen ist. Die frei liegende Endpartie 29 des Ausläufers 26 bzw. 27 weist eine Bohrung 28 auf. Diese Bohrung 28 verläuft von der Stirnfläche 49 der frei liegenden Endpartie 29 des Führungsausläufers 26 bzw. 27 gegen die Basisplatte 1 hin, und zwar schräg aufwärts, sodass die zweite Mündung 48 dieser Bohrung 28 in der äusseren Oberfläche des gebogenen Führungsausläufers 26 bzw. 27 liegt. Die Bohrung 28 im ventralen Ausläufer 26 ist vorgesehen für die Durchführung des Seils 22, welches vom dorsalen Zinken 17 herangeführt wird. Die Bohrung 28 im dorsalen Ausläufer 27 ist für die Durchführung des Seils 21 vorgesehen, welches vom ventralen Zinken 16 herangeführt wird.

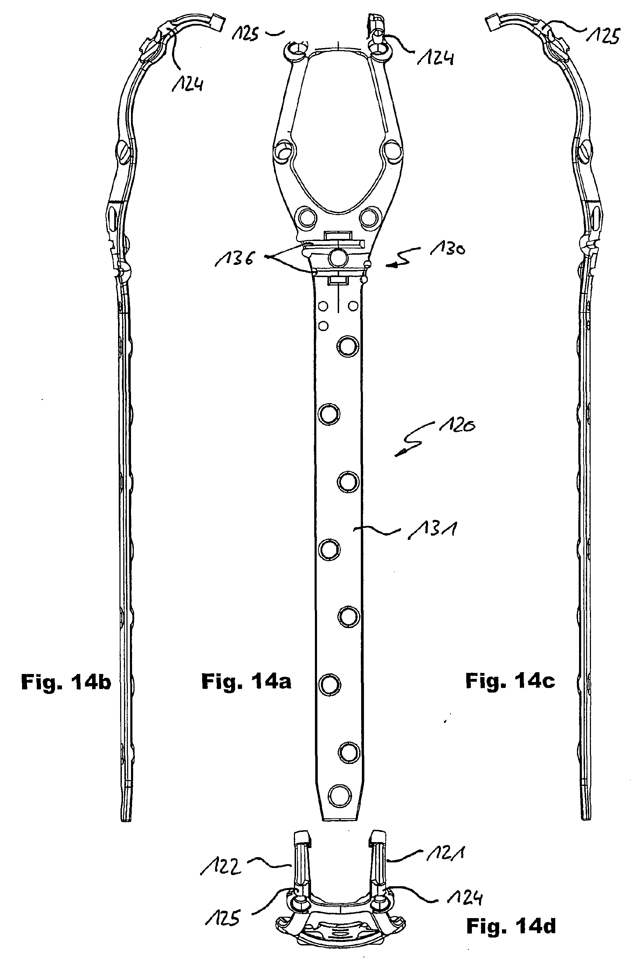

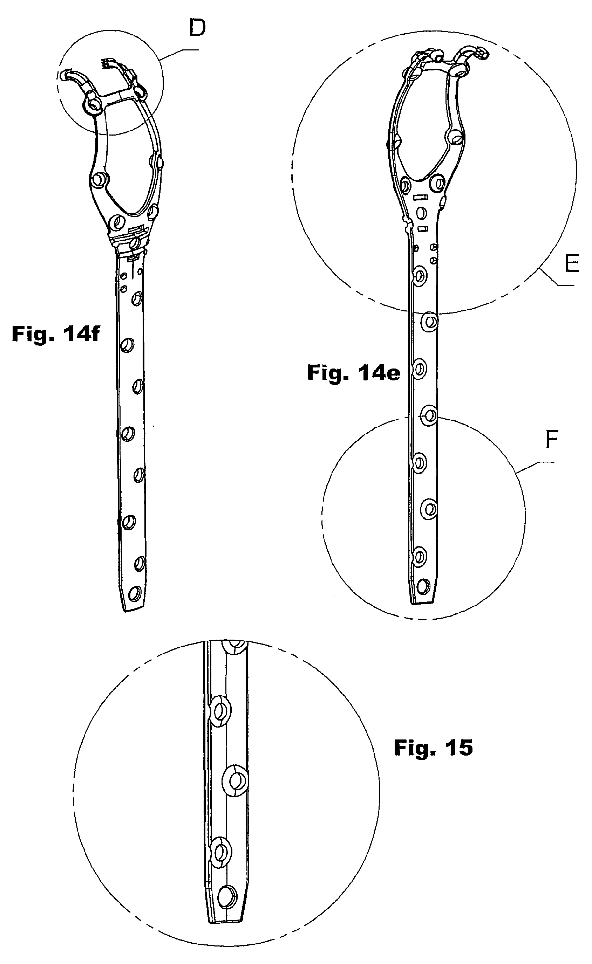

Insbesondere bei Ausführungsformen der erfindungsgemässen Implantate, die bei der ETO eingesetzt werden, hat es sich, wie im weiteren noch genauer erläutert wird als vorteilhaft erwiesen, die flexiblen, länglichen Glieder direkt nach Verlassen der Zinken über der Fossa trochanterica (15) zu kreuzen, von dort zum medialen Umfang des Trochantermassiv (Calcar femoris) zu führen und dort nochmals zu überkreuzen und dann annähernd senkrecht zur Längsachse des Schaftes zurück zur Klemmvorrichtung der Basisplatte zu führen. Auf die Führungsausläufer 26 bzw. 27 kann daher, wie es bei den Basisplatten der Figuren 14, 17 und 18 angedeutet ist, verzichtet werden.

Der untere Abschnitt 3 der Basisplatte 1 schliesst sich an den unteren Rand 38 des Hauptteils 5 des oberen Plattenabschnittes 4 an, und zwar unterhalb der Klemmvorrichtung 30. Der untere Abschnitt 3 der Basisplatte 1 ist mit dem oberen Plattenabschnitt 4 einstückig.

Ähnlich wie der obere Abschnitt 4 der Basisplatte 1 weist der untere Abschnitt 3 der Basisplatte 1 einen flächenhaften Grundkörper bzw. Hauptteil auf, welcher länglich gewölbt ausgeführt ist. Die Längsachse dieser länglichen Wölbung fällt mit der Längsachse der Krümmung der Aussenfläche des Femur 2 zusammen oder sie läuft zumindest parallel dazu. Die Krümmung dieses unteren Abschnittes 3 der Basisplatte 1 soll der Krümmung der Oberfläche jenes Abschnittes des Schenkelknochens 2 entsprechen, welchem dieser Abschnitt 3 der Basisplatte 1 zugeordnet werden soll. Es versteht sich, dass die Krümmung dieses Abschnittes 3 der Basisplatte 1 entlang seiner praktisch vertikal verlaufenden Längsachse veränderlich sein kann, je nach dem, wie sich die horizontal verlaufende Krümmung der Oberfläche dieses Abschnittes des Schenkelknochens 2 in seiner Längsrichtung ändert.

Eine erste Hauptaufgabe des unteren Abschnittes 3 der Basisplatte 1 besteht darin, die Abduktorenkräfte von den Zinken lateral und von den Kabelseilen medial kommend zu neutralisieren. Eine zweite Hauptaufgabe des unteren Abschnittes 3 der Basisplatte 1 ist es, die Rotationsstabilität der Konstruktion für den Fall sicherzustellen, dass der Vektor des Abduktorenzugs nicht mit der Längsachse des Femur 2 identisch ist.

Der distale Abschnitt 3 der Basisplatte 1 weist drei Bereiche 81, 82 und 83 auf (Fig. 1). Der obere Bereich 81 des distalen Abschnittes 3 der Basisplatte 1 schliesst sich an den unteren Rand 38 des proximalen Bereichs 4 der Basisplatte 1 an. Zwischen diesen ist eine erste Nut 67 in der Aussenseite des distalen Abschnitts 3 der Basisplatte 1 ausgeführt, welche praktisch senkrecht zur Längsachse dieses distalen Abschnittes 3 der Basisplatte 1 verläuft. In diesem oberen Bereich 81 des distalen Abschnitts 3 der Basisplatte 1 sind zwei Langlöcher 68 und 69 ausgeführt, welche sich in einem Abstand voneinander befinden. Die Längsachse dieser Langlöcher 68 und 69 verläuft vertikal. Zwischen dem oberen Bereich 81 und dem mittleren Bereich 82 des distalen Abschnittes 3 der Basisplatte 1 ist eine zweite Nut 77 in der Aussenseite des distalen Abschnittes 3 der Basisplatte 1 ausgeführt. Im mittleren Bereich 82 des distalen Abschnitts 3 der Basisplatte 1 sind ebenfalls zwei Löcher 72 und 73 ausgeführt, welche allerdings eine kreisförmige Kontur haben können. Je eines dieser kreisförmigen Löcher 72 bzw. 73 liegt unterhalb eines der Langlöcher 68 bzw. 69 im oberen Bereich 81. Im unteren Bereich 83 des distalen Abschnitts 3 der Basisplatte 1 ist ein Loch 74 mittig ausgeführt. Die Fixation des distalen Abschnitts 3 der Basisplatte 1 am proximalen lateralen Femur 2 kann mittels Schraubelementen erfolgen, welche durch die Löcher 68 und 69 im oberen Bereich 81 des distalen Abschnitts 3 der Basisplatte 1 oder/und durch die Löcher 72 und 73 im mittleren Bereich 82 des distalen Abschnitts 3 der Basisplatte 1 hindurchgehen.

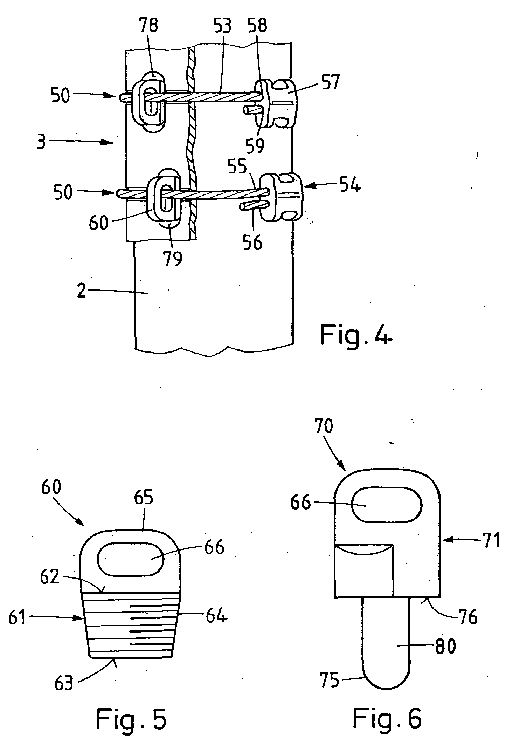

Fig. 4 zeigt einen Ausschnitt aus dem unteren Abschnitt 3 der Basisplatte 1, welcher mit Hilfe von Fixiervorrichtungen bzw. Kabel-Cerclagen 50 am Knochen 2 befestigt ist. Die Fixiervorrichtung 50 umfasst ein längliches und biegsames Glied 53 (Fig. 1 und 4), welches um den Femur 2 herum ringförmig gelegt ist. Dieses Glied 53 ist zweckmässigerweise ein Seil. Das Kabel 53 liegt in einer der Nuten 67 bzw. 77 im unteren Abschnitt 3 der Basisplatte 1 und der übrige Teil desselben umgibt den Femur 2. Das Kabel 53 hat Endpartien 55 bzw. 56. Die Fixiervorrichtung 50 umfasst ferner ein Klemmmittel 54, welches die Endpartien 55 und 56 des um den Femur 2 herumgelegen Seils 53 zusammenhalten kann. Das Klemmmittel 54 hat einen Grundkörper 57 aus einem Material, welches zwar formstabil ist, welches sich jedoch unter der Anwendung einer beträchtlichen mechanischen Kraft, beispielsweise ausgeübt durch eine Zange, dennoch deformieren lässt. Im Grundkörper 57 des Klemmmittels 54 sind zwei durchgehende Bohrungen 58 und 59 ausgeführt, welche sich in der Längsrichtung des Klemmmittels 54 erstrecken. Diese Bohrungen 58 und 59 liegen nebeneinander und sie verlaufen praktisch parallel zueinander. Der Durchmesser dieser Bohrungen 58 und 59 ist so gewählt, dass eines der Seilenden 55 bzw. 56 durch eine der Bohrungen 58 bzw. 59 gerade hindurchgehen kann. Nach dem der Grundkörper 57 des Klemmmittels 54, wie erwähnt, deformiert, insbesondere zusammengedrückt worden ist, wird auch die Form des Querschnitts der Löcher 58 und 59 geändert, insbesondere zusammengedrückt. Dadurch werden die Seilenden 55 und 56 in diesen Bohrungen 58 und 59 festgeklemmt und durch das Klemmmittel 54 festgehalten.

Durch die primäre Schraubenbelegung der Langlöcher 68 und 69 und/oder 72 und 73 kann der distale Abschnitt 3 der Basisplatte 1 temporär am Femur 2 fixiert werden. Die Belegung der Löcher 72 und 73 im mittleren Bereich 82 des distalen Abschnitts 3 der Basisplatte 1 erfolgt wahlweise mit Zugschrauben oder mit winkelstabilen Schrauben. Die zentral distal im unteren Bereich 83 des distalen Abschnitts 3 der Basisplatte 1 liegende Öffnung 74 dient dem Einsatz eines Plattenapproximators für die Distalisierung der gesamten Zuggurtungskonstruktion. Anschliessend werden eine oder zwei Fixiervorrichtungen 50 angebracht, wobei das Kabel 53 der jeweiligen Fixiervorrichtung 50 in eine der Nuten 67 bzw. 77 zu liegen kommt.

Der Grundkörper des unteren Abschnittes 3 der Basisplatte 1 ist verhältnismässig dünn, sodass die Nuten 67 und 77 in diesem nur wenig tief sein können. Dies kann dazu führen, dass die Kabel 53 unter Umständen die Nut 67 bzw. 77 verlassen, was zur Folge haben kann, dass sich die Fixiervorrichtung 50 in der Längsrichtung des Knochens 2 verschiebt. Um dies zu verhindern, weist der Grundkörper des unteren Abschnittes 3 der Basisplatte 1 übereinander liegende Langlöcher 78 und 79 auf, deren Längsachse praktisch vertikal verläuft. Es ist ein Halter 60 vorgesehen, welcher zur Positionierung des Seils 53 in der Längsrichtung des unteren Abschnittes 3 der Basisplatte 1 am Femur 2 bestimmt und ausgeführt ist.

Fig. 5 zeigt in einer Seitenansicht eine erste Ausführung eines solchen Halters 60 der Fixiervorrichtung 50. Dieser Halter 60 weist einen Grundkörper 61 auf, welcher zum Einsetzen in eines der Langlöcher 78 bzw. 79 im unteren Abschnitt 3 der Basisplatte 1 bestimmt und ausgeführt ist. Der Haltergrundkörper 61 ist etwa konusförmig, wobei dieser Konus 61 eine obere Stirnfläche 62 und eine untere Stirnfläche 63 hat. Die untere Stirnfläche 63 hat einen kleineren Durchmesser als die obere Stirnfläche 62. Der Abstand zwischen den Stirnflächen 62 und 63 kann der Dicke des unteren Abschnittes 3 der Basisplatte 1 gleichen oder er kann etwas grösser als diese sein. Der Mantel 64 des Konus 61 ist mit einem Gewinde oder zumindest mit Vorsprüngen versehen. Von der grösseren Stirnfläche 62 des Konus 61 steht eine Öse 65 ab, welche mit dem Grundkörper 61 des Ringhalters 60 einstückig ist. In der Öse 65 ist eine Öffnung 66 ausgeführt, durch welche jener Abschnitt des Seils 53 hindurchgehen kann, der sich zwischen den Endpartien 55 und 56 desselben erstreckt. Zweckmässigerweise ist die Öffnung 66 als ein Langloch ausgeführt, wobei die Längsachse dieses Langloches 66 im Ringhalter 60 etwa parallel zur oberen Stirnfläche 62 des Ringhalters 60 verläuft. Zunächst wird das Seil 53 durch die Öffnung 66 in der Öse 65 des Ringhalters 60 hindurchgeführt. Dann wird der Ringhalter 60 in eine der Öffnungen 78 bzw. 79 im unteren Abschnitt 3 der Basisplatte 1 eingesetzt. Danach wird, das Seil 53 um den Knochen 2 herumgelegt und die Seilenden 55 und 56 durch die Bohrungen 58 und 59 im Klemmmittel 54 hindurch gesteckt. Der Grundkörper dieses Klemmmittels 54 wird unter Spannung der Seilenden 55 und 56 deformiert, insbesondere zusammengedrückt. Als vorteilhaft gilt bei dieser Ausführung des Ringführers 60, dass der Ringführer 60 im unteren Abschnitt 3 der Basisplatte 1 festsitzt und dass es daher den Knochen 2 nicht oder nur minimal berührt. Unter bestimmten Umständen ist jedoch eine zweite Ausführung des Ringhalters 70 zweckmässig, welche in Fig. 6 in einer Seitenansicht dargestellt ist. Dieser Ringhalter 70 weist einen im Wesentlichen flächenhaften Grundkörper 71 auf, dessen Querschnitt ovalförmig sein kann. Folglich kann dieser Grundkörper 71 durch eine der ovalen Öffnungen 78 bzw. 79 im unteren Abschnitt 3 der Basisplatte 1 hindurchgehen. Im oberen Bereich des Ringführers 70 ist die bereits beschriebene Öffnung 66 zur Durchführung des Seils 53 vorhanden. Von der unteren Stirnfläche 76 des Grundkörpers des Ringhalters 70 steht ein Zapfen 80 ab. Die freie Endpartie 75 dieses Zapfens 80 ist abgerundet. Damit dieser Ringführer 70 eingesetzt werden kann, muss allerdings zunächst eine Vertiefung (nicht dargestellt) im Knochen 2 gebohrt werden, in welche der Zapfen 80 zu liegen kommt, nachdem dieser Ringhalter 70 in eine der Langlöcher 78 bzw. 79 eingesetzt worden ist.

Die beschriebene Basisplatte 1, welche am proximalen Femur 2 fixiert ist, die Zinken 16 und 17 sowie die Seile 21 und 22 bilden ein Zuggurtungskonstrukt, welches das Trochanter-Fragment 9 sowohl lateral als auch cranial und medial umfasst, damit dieses an anatomischer Position gehalten wird.

Der osteotomierte/frakturierte Trochanter major 9 wird manuell oder mit einer Klemme fixiert und mit einem rohrförmigen Kabelpasser (nicht dargestellt) von dorsal-medial umfahren. Dabei wird von medial-dorsal kommend die Sehnenplatte direkt über der Spitze des Trochanter-Fragments 9 in der ventralen Hälfte durchstossen. Über den so vorgelegten Kabelpasser wird das Kabelseil 21 des ventralen Zinkens 16 hindurchgeführt, sodass das Seil 21 von ventral über die Trochanterspitze verläuft und nach dorsal-medial zieht. Von dort wird das Seil 21 weiter dorsalseitig am proximalen Femur 2 entlang an die Basisplatte 1 zurückgeführt. Anschliessend wird das zweite Kabelseil 22 entsprechend von dorsal über die Trochanterspitze nach ventral-medial geführt und dann weiter ventral am proximalen Femur 8 an die Basisplatte 1 geleitet. Unter Zug an beiden freien Kabelseilenden 142 und unter gleichzeitigem leichten Druck auf den proximalen Bereich 4 der Basisplatte 1 nach medial werden die Zinken 16 und 17 durch die Sehnenplatte des Musculus gluteus medius geschoben, bis die vorgebogene Krümmung der Zinken 16 bzw. 17 die Trochanterspitze umfasst. Nun kann durch gleichzeitigen Zug an den freien Enden 142 der beiden Kabelseile 21 und 22 und durch den gleichzeitig ausgeübten Druck auf die Basisplatte 1 die Reposition des Trochanter-Fragments 9 probehalber ausgeführt werden. Bei dieser Reposition sollte das Bein vorteilhafterweise in Abduktion und Aussenrotation gehalten werden. Dadurch wird die Spannung des Musculus gluteus medius verringert und die Reposition vereinfacht. Bei anatomischer Reposition sollte die Basisplatte 1 distal des Tuberculum innominatum 10 sowie der ventrale Plattenschenkel 6 ventral und der dorsale Plattenschenkel 7 dorsal des Tuberculum innominatum 10 liegen. Ist die Platte 1 in der Höhe nicht korrekt positioniert, insbesondere zu hoch, d.h. auf dem Tuberculum innominatum 10 liegend, sollten die Zinken 16 bzw. 17 nachgebogen werden, damit die optimale Lage erreicht wird. Bei korrekter Plattenlage können nun die beiden Langlöcher 68 und 69 im distalen Bereich 3 der Basisplatte 1 mit Schrauben (nicht dargestellt) am distalen Langlochende belegt werden. Anschliessend werden die freien Enden 142 der Seile 21 bzw. 22 durch den jeweiligen Führungsausläufer 26 bzw. 27 der Basisplatte 1 an die Klemmvorrichtung 30 heran und unter der Klemmplatte 31 hindurchgeführt. Dabei werden die Enden 142 der Seile 21 bzw. 22 so unter die Klemmplatte 31 eingelegt, dass beim späteren Anziehen der Feingewindeschraube 32 der Klemmvorrichtung 30 (Rechtsgewinde) tendenziell die Seile 21 bzw. 22 zusätzlich gespannt werden.

Mit einem doppelseitigen Kabelspanner (nicht dargestellt) werden nun die Kabelseile 21 und 22 gespannt. Dabei verblocken sich die Endpartien 19 der flexiblen Plattenzinken 16 bzw. 17 an der Kreuzung der Kabelseile 21 und 22, d.h. über der Fossa trochanterica 15. Unter weiterem Zug passen sich die flexiblen Zinken 16 bzw. 17 der Basisplatte 1 der Kontur der cranialen Zirkumferenz des Trochanter major 9 an und ziehen diesen an anatomischer Position fest. Die Kabelseile 21 bzw. 22 werden soweit gespannt, dass genügend hoher Zug erreicht wird, ohne den Trochanter major 9 zu deformieren oder aus seiner reponierten Lage zu luxieren.

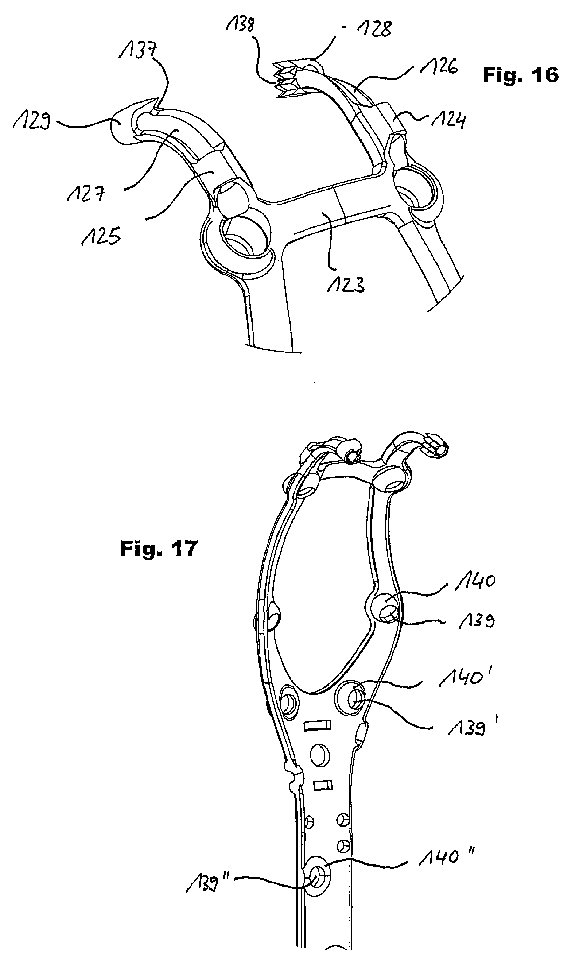

In der Figur 16 ist eine bevorzugte Ausgestaltungsform der Endpartien respektive der Verdickungen 128, 129 der flexiblen Plattenzinken dargestellt, die durch einen formschlüssigen Eingriff von korrespondierenden verzahnten Innenflächen 137, 138 die Verblockung der Zinken unterstützt.

Für die weiteren Schritte bestehen gemäss bevorzugten Ausführungsformen mehrere Optionen:
Option 1: Belassen des doppelseitigen Kabelspanners, Belegen von mindestens zwei Optionen der Löcher 68 und 69 im distalen Bereich 3 der Basisplattel der beiden Löcher 72 bzw. 73 im mittleren Teil 82 des distalen Bereichs 3 der Basisplatte 1 mit Schrauben 36. Die Schraubenlöcher 355 und 356 sind obligatorisch mit Schrauben 36 zu belegen. Von den vier möglichen winkelstabilen Schrauben 36 im proximalen, flexiblen Abschnitt 4 der Basisplatte 1 sind wenigsten zwei zu belegen. Im Anschluss daran sollte nochmals der Kabelspanner nachgezogen und so die definitive Spannung der Kabelseile 21 und 22 eingerichtet werden. Zum Schluss wird die Feingewindeschraube 32 der Klemmvorrichtung angezogen und die überstehenden Kabelseilenden 142 werden abgeschnitten.
Option 2: Fixieren der Kabelseile 21 und 22 durch Andrehen der Feingewindeschraube 32 der Klemmvorrichtung 30 und geringgradiges Öffnen der Schrauben 36 in den Langlöchern 68 und 69. Installieren eines Approximators am distalsten Loch 74 der Basisplatte 1, womit die gesamte Platten-Kabelseil Konstruktion distalisiert und dadurch ein nochmals höherer Anpressdruck über der Trochanter-Osteotomie/-Fraktur erzeugt wird. Festdrehen der Schrauben in den Langlöchern 68 und 69 und Belegen der Schraubenlöcher 72 und 73, wie in Option 1 beschrieben. Die Schraubenlöcher 355 und 356 sind obligatorisch zu belegen. Schliesslich werden die Kabelseilenden 142 abgeschnitten.
Option 3: Die Fixation der Basisplatte 1 am proximalen Femur 2 kann ausschliesslich oder in Kombination mit Kabelseilen 21 und 22 über die dafür vorgesehenen Nuten bzw. Vertiefungen 67 und 77 im distalen Abschnitt 3 der Basisplatte 1 erfolgen. Diese Variante wird angewandt, wenn eine Schädigung des Prothesenschafts durch ein eventuell mögliches Anbohren beim Einbringen der Schrauben 36 unbedingt vermieden werden soll. Die Fixation erfolgt dann wie in Option 2 nicht durch Belegung der Schrauben 36, sondern üblicherweise mittels Applikation Fixiervorrichtungen 50. Die Schraubenlöcher 355 und 356 sind obligatorisch zu belegen.
Option 4: Die Kabelseile 21 und 22 können nach dem Austritt aus den Zinken 16 bzw. 17 und der Überkreuzung an der Fossa trochanterica 15 zusätzlich unterhalb des Trochanter minor medial um den proximalen Femur 8 geführt und dann zur Basisplatte zurückgeführt und dort fixiert werden. Dadurch wird der Zugvektor der Kabelseile 21 und 22 nach caudal-medial verlegt und so ein mögliches Luxieren der Zuggurtungskonstruktion über die Osteotomie-Ebene OF nach lateral verhindert. Die Schraubenlöcher 355 und 356 sind obligatorisch zu belegen.

Gemäss weiterer vorteilhafter Ausführungsformen der oben genannten Erfindung weisen die Implantate für die Refixation des osteotomierten oder frakturierten Trochanter major, mindestens eine am proximalen Femur fixierbare Platte auf, welche kraftschlüssig am Femur gehalten wird. Die Platte weist mindestens einen proximalen Ausläufer auf, der mit einer gegenüberliegenden Seite des Trochanter major mit mindestens einem Zügel verbunden ist, der zur Platte zurück läuft, dort wie oben beschrieben fixiert wird und mit dem mindestens einen proximalen Ausläufer ein "geschlossenes System" bildet. Der mindestens eine proximale Ausläufer und der mindestens eine Zügel bilden zusammen eine Zuggurtungskonstruktion, die über den Trochanter major 9 verläuft.

Insbesondere der untere Abschnitt der Basisplatte, im Folgenden auch Plattenschaft, oder kurz Schaft genannt, erfährt in bevorzugten Ausführungsformen Modifikationen, wie sie nachfolgend kurz dargelegt sind.

Die Länge des Schafts kann variiert werden, wobei die maximale Länge eine Femurlänge beträgt. Erfindungsgemässe Implantate mit kurzem Schaft sind besonders geeignet für traditionelle Osteotomien des Trochanter major, bei denen nur die Spitze des Trochanter major schräg abgesetzt wird. Eine mittlere Schaftlänge hat sich bei der Ganz-Osteotomie bewährt, und Implantate mit langem Schaft sind insbesondere geeignet bei der so genannten "extended osteotomy". Die langen Schaftversionen lassen sich bei allen Arten der Osteotomie einsetzen, wobei sie für die traditionelle und die Ganz-Osteotomie zwar operativ-technisch möglich, aber oft schlicht nicht nötig sind.

Um insbesondere bei der Ganz-Ostetomie zu verhindern, dass das Implantat aufträgt und stört, kann der Schaft fast vollständig reduziert sein, so dass die Basisplatte zum Beispiel nur durch zwei Schrauben am Femur fixiert wird.

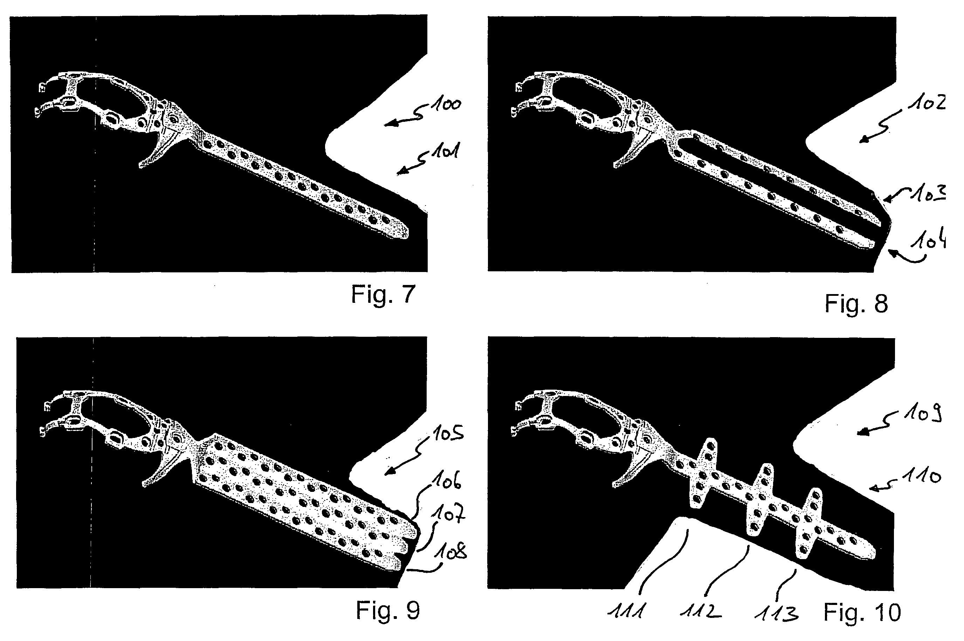

In der Figur 7 ist eine weitere Ausführungsform eines erfindungsgemässen Implantats dargestellt, bei dem der untere Abschnitt einer Basisplatte 100 als langer schmaler Plattenschaft 101 ausgebildet ist, der sich, wie im folgenden noch beschrieben wird, beim Implantieren gut zum Unterschieben unter den M. vastus lateralis eignet. Gemäss einer vorteilhaften, in den Figuren nicht dargestellten Ausführungsform ist der Schaft wellenförmig ausgebildet, so dass er im Aufbau einer Snake Plate entspricht, wie sie von der Firma Icotec bekannt ist. Beim wellenförmigen Schaft sind die Aufnahmeöffnungen für die Schrauben nicht in einer Linie angeordnet, so dass keine Sollbruchlinie generiert wird.

In der Figur 8 ist eine weitere vorteilhafte Ausführungsform gezeigt, bei der der untere Abschnitt der Basisplatte als gegabelter Plattenschaft 102 mit zwei im Wesentlichen parallelen voneinander beabstandeten Längsflügeln 103, 104 ausgebildet ist. Die Längsflügel 103, 104 greifen unterhalb der Klemmvorrichtung am distalen Ende des oberen Abschnitts an. In der vorliegenden Ausführungsform kann jeder einzelne Flügel schmäler ausgebildet sein als der Schaft bei einflügligen Varianten (wie zum Beispiel gemäss Fig. 7), ohne die Stabilität zu beeinträchtigen. Die schmalen Längsflügel haben den Vorteil, dass sie sich bei der Operation noch leichter unter den M. vastus lateralis schieben lassen. Der Ursprung des M. vastus lateralis muss entsprechend beim Implantieren nicht oder nur wenig abgelöst werden wenn das Implantat mit der zweiflügeligen Platte 102 von proximal eingeführt wird. Die Flügel 103, 104 können in weiteren, nicht dargestellten Ausführungsformen starr oder gelenkig miteinander verbunden sein.

Eine weitere vorteilhafte Ausführungsform der Basisplatte des erfindungsgemässen Implantats ist in der Figur 9 dargestellt. Der untere Abschnitt der Basisplatte ist hierbei als breiter Schaft 105 ausgebildet, der eine noch bessere Fixation am Knochen erlaubt. Die breite Fixation verbessert den Widerstand im Vergleich zu einer schmalen Platte gegenüber der Hebelwirkung bei Rotationsmomenten. Ein weiterer Vorteil des breiten Schaftes kommt beim Einsatz bei der so genannten Extended Trochanteric Osteotomy (ETO) zum Tragen. Der Schaft 105 ümfasst drei Segmente 106, 107, 108, wobei Anteile der dorsalen und ventralen Stegmente 106 und 108 über die Osteotomie-Linie der Extended Trochanteric Osteotomy ragen, so dass der Schaft 105 jenseits (Bereich b) und diesseits (Bereich a) der Osteotomie-Linie vorzugsweise mittels Schrauben fixiert werden kann. Der plattenförmige Schaft 105 ist beiderseits der Osteotomie-Linie mit Schrauben fixiert, das heisst er wird am distalen Fragment des Femurs und im Osteotomie/Trochanter-Fragment fixiert und verhindert damit das Proximalwandern des Osteotomie-/Trochanter-Fragments.

Ein ähnlich vorteilhafter Effekt bei der Extended Trochanteric Osteotomy lässt sich mit einem Implantat mit einer Basisplatte 109 gemäss einer weiteren Ausführungsform, wie sie in der Figur 10 gezeigt ist, erzielen. Die Basisplatte 109 ist als langer schmaler Plattenschaft 110 ausgebildet, von dem drei Paar Lateralflügel 111, 112, 113 im wesentlichen senkrecht abstehen. Es ist für den Fachmann offensichtlich, dass die Seitenflügel 111, 112, 113, die in der Abbildung annähernd in einer Ebene mit dem Schaft 110 dargestellt sind, intraoperativ an die Geometrie des Femurs angepasst werden und nach dem Fixieren aus dieser gemeinsamen Ebene ausgelenkt sind. Der mit Lateralflügeln 111, 112, 113 versehene Schaft 110 ist wiederum beiderseits der Osteotomie-Linie sowohl im distalen Fragment des Femurs als auch im Osteotomie/Trochanter-Fragment mit Schrauben fixiert und verhindert sehr effektiv das Proximalwandern des Osteotomie/Trochanter-Fragments.

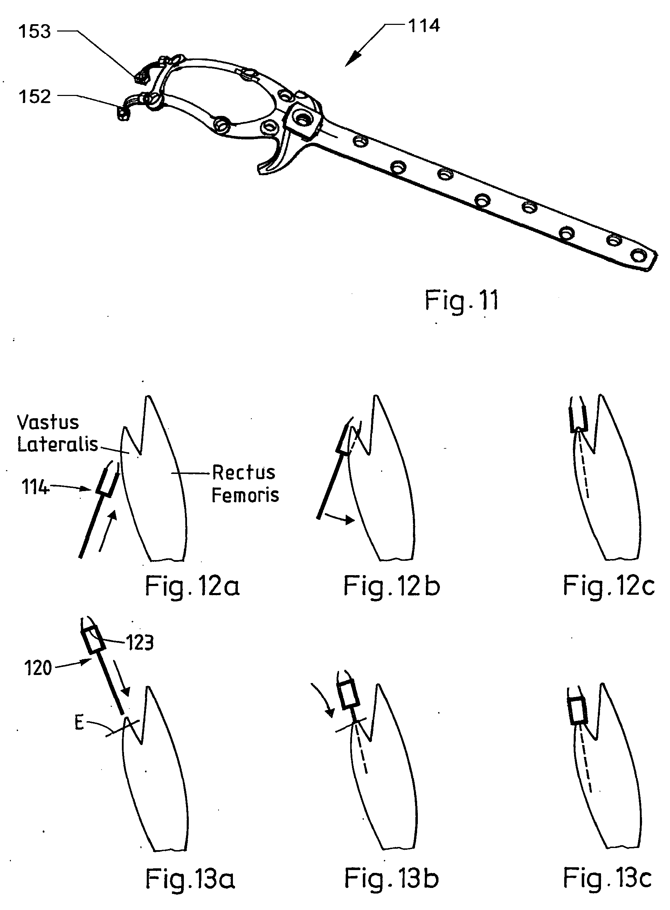

In der Figur 11 ist eine Basisplatte 114 eines erfindungsgemässen Implantats in einer weiteren Ausführungsform dargestellt, bei dem der obere Abschnitt der Basisplatte ohne Quersteg ausgebildet ist. Die Basisplatte 114 kann dadurch, wie in den Figuren 12 a bis 12c skizziert ist, insbesondere bei der minimal-invasiven Implantation (vor allem bei Ganz-Osteotomie), von distal des Ursprungs des M. vastus lateralis mit den oberen beiden Plattenzinken 115, 116 anterior und posterior des Muskelursprungs herumgeführt werden, ohne dass dieser abgetrennt werden muss. Die Basisplatte 114 kann herstellerseitig bereits ohne Quersteg gefertigt sein, oder eine Platte mit einer Querstegkonstruktion ist mit geeigneten Rillen oder ähnlichen Sollbruchstellen versehen, so dass der Quersteg vom Operateur intraoperativ bei bedarf entfernt werden kann.

In den Figuren 13a bis13c ist ein Beispiel für das operative Einsetzen einer Basisplatte 120 mit Quersteg 123 am oberen Abschnitt, wie sie zum Beispiel in der Figur 14 gezeigt ist, dargestellt. Die Basisplatte 123 mit Quersteg wird von oben/proximal nach Einschneiden eines kurzen Anteils des Ursprungs des M. vastus lateralis (in der Figur 13a durch einen kurzen schwarzen Querstrich E angedeutet) unter diesen eingeschoben.

Anhand der Basisplatte 120, wie sie in den Figuren 14 bis 19 dargestellt ist, soll im Folgenden auf weitere vorteilhafte Merkmale der vorliegenden Erfindung eingegangen werden, die sich, soweit nicht ausdrücklich erwähnt, auch bei anderen Ausführungsformen der erfindungsgemässen Implantate realisieren lassen.

Aus den verschiedenen Ansichten der Figur 14 und insbesondere aus der Detailvergrösserung D, wie sie in der Figur 16 dargestellt ist, ist gut erkennbar, dass die Endabschnitte der Zügel nicht nur an der ersten Endpartie, das heisst an der Spitze, der Zinken 121, 122, sondern auch an der entgegengesetzt liegenden Endpartie, also der Zinkenbasis, angreifen können. Dazu ist an der Basis der Zinken 121, 122 jeweils eine Verdickung 123, 124 angeordnet, die wie bereits oben für die Verdickungen an der Spritze der Zinken der Ausführungsform gemäss der Figuren 1 bis 3 beschreiben wurde, mit einer Bohrung zur Durchführung der biegsamen, länglichen Glieder versehen ist. Die Glieder, vorzugsweise Kabelseile die in den Figuren 14 bis 18 nicht eingezeichnet sind, sind wiederum vorzugsweise an ihren rückwärts ragenden Endabschnitten mit Kappen versehen, welche verhindern, dass der jeweilige Seilendabschnitt durch die Bohrung in der Verdickung 124, 125 gezogen werden kann. die Zinken sind oberseitig mit einer Führungsnut 126, 127 versehen, die das Kabelseil entlang des Zinkens 121, 122 zur Längsbohrung in der Verdickung 128, 129 an der Zinkenspitze führt und gegen ein Dislozieren unter Zugbelastung sichert.

Die in der Figur 12 abgebildete Basisplatte 120 mit einem langen Schaft 130 hat sich als vorteilhaft bei der Extended Trochanteric Osteotomy (ETO) erwiesen, bei der ein alternativer Verlauf der Zügel, vorzugsweise in Form von Kabelseilen, vorgesehen ist. Die Basisplatte 120 weist keine Führungsausläufer zur Aufnahme der zurückgeführten Kabelseile auf. Solche Führungsausläufer sind bei den oben beschriebenen Ausführungsformen (gem. Fig. 2) an den Seitenrändern des Hauptteils 5 der Basisplatte 1 zwischen der oberen horizontal verlaufenden Randpartie 39 des Hauptteils 5 und der unteren horizontal verlaufenden Randpartie 38 desselben auf Höhe der Klammervorrichtung 30 angeordnet. Bei der Basisplatte gemäss Figur 14 kann auf die Führungsausläufer zur Aufnahme der zurückgeführten Kabelseile, die insbesondere beim minimal-invasiven Implantieren hinderlich sein können, verzichtet werden, da die Kabelseile um das Trochantermassiv herum horizontal zur Basisplatte zurückkommen. Die beiden Kabelseile verlaufen direkt nach Verlassen der Zinken gekreuzt über der Fossa trochanterica und von dort zum medialen Umfang des Trochantermassiv (Calcar femoris). Dort überkreuzen sich die Kabelseile erneut und gehen dann annähernd senkrecht zur Längsachse des Schaftes zurück zur Klemmvorrichtung 130 der Basisplatte 120. Bei der ETO wird damit wirksam verhindert, dass die Kabelseile in die Osteotomie hineinrutschen und keinen Halt am Femur finden.

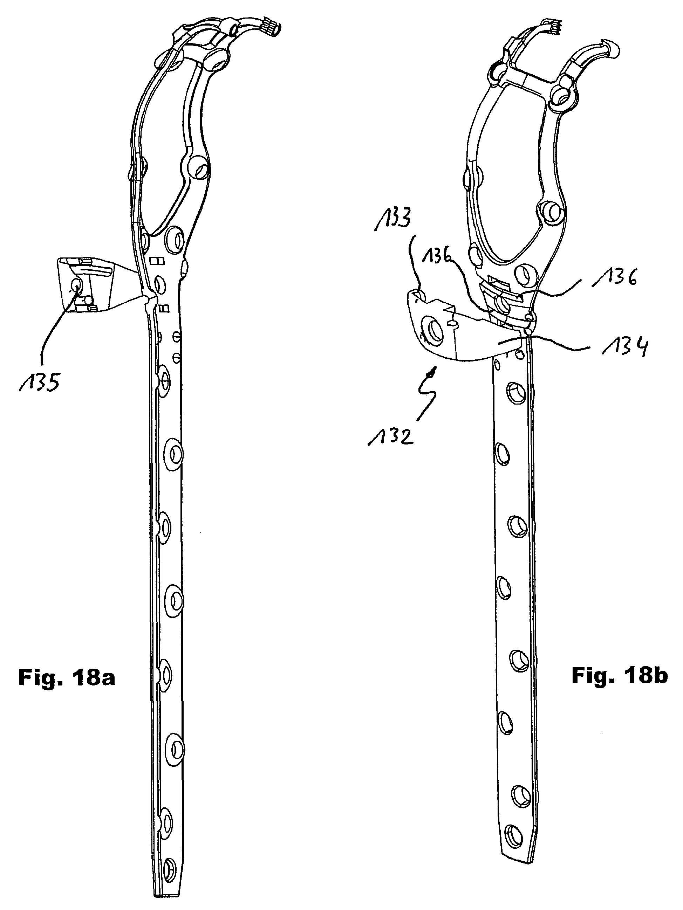

Im Falle einer Standard-Osteotomie oder erschwerter Verhältnisse für die Kabelseilführung medial am Femur entlang (Calcar femoris) können die Kabelseile optional nach der ersten Kreuzung über der Fossa trochanterica auch wie bei den vorgängig beschriebenen Ausführungsformen direkt zur Platte zurückgeführt werden. Zur besseren Führung der Kabelseile im Endbereich kann bei diesen Fällen, eine in der Figur 18 dargestellte Klemmplatte oder Kompressionsplatte 132 mit seitlichen Ausläufern 133, 134 zum Einsatz kommen. Eine Durchführöffnung 135 in der Spitze des Ausläufers 133, 134 nimmt jeweils ein Kabelseil auf und führt es zum eigentlichen Klemmbereich im Zentrum der Kompressionsplatte. Die Klemmung der Kabelseile und die Ausgestaltung von Nuten oder Vertiefungen 136 zur Verringerung der Bauhöhe des Implantats im Bereich der Klemmvorrichtung unterscheidet sich dabei nicht wesentlich von den vorgängig beschriebenen Ausführungsformen.

Für die Standard-Osteotomie und die Ganz-Osteotomie wird, im Gegensatz zur ETO, vorteilhafter Weise ein Implantat eingesetzt, bei dem die Basisplatte in allen wesentlichen Merkmalen mit der Basisplatte gemäss der Figur 18 übereinstimmt, der Schaft aber stark verkürzt ausgebildet ist.

Gemäss weiterer bevorzugter Ausführungsformen wird die Basisplatte mit wenigen mm oder cm Abstand zum Knochen montiert. Dieses no contact oder low contact-Design hat den Vorteil, dass Knochen, Knochenhaut oder auch Muskel unter der Platte nicht kompromittiert werden. Da die Durchblutung des Knochens, der Knochenhaut und oder des Muskels unter der Platte nicht oder nur wenig beeinträchtigt werden, sinkt das Risiko von Nekrosen von Knochen und/oder Muskeln unter der Platte, die Knochen/Muskeln bleiben vital und intakt, was wiederum bedeutet, dass die Platte stabil fixiert bleibt. Ohne Nekrosen wird bakteriellen Infektionen kein Nährboden geboten und eine intakte Durchblutung des Knochens und/oder Muskels bedeutet, dass die Heilung der Osteotomie, zumindest in den Bereichen in denen die Osteotomie-Linie in der Nähe der Basisplatte verläuft, nicht negativ beeinflusst wird.

Zur Umsetzung des no contact- oder low contact-Designs sind vorzugsweise eine Mehrzahl von Abstandsnocken an der, dem Knochen zugewandten, Seite der Basisplatte angeordnet oder es sind Abstandshalter 140 um die Schraubenlöcher 139 herum an der gleichen Seite der Basisplatte angeordnet, wie es in der Figur 17 angedeutet ist.

Werden winkelstabile Schraubensysteme zur Kompression des Schaftes oder der Basisplatte am Femur eingesetzt oder stehen andere geeignete Gewindelöcher zur Verfügung, so lässt sich der Abstand zwischen Platte und Knochen auch mittels einschraubbarer Bolzen einstellen, wie sie zum Beispiel vom NCB-System der Firma ZIMMER als Abstandshalter bekannt sind. Die Bolzen werden vor Implantation der Platte in die Schrauben/Bolzenlöcher eingedreht und ermöglichen je nach Bolzentyp das Einstellen variable Abstände, und lassen sich nach der Implantation der Platte auf Wunsch auch wieder entfernen.

Zur Fixation des Plattenschaftes am Knochen kann allgemein gesagt werden, dass sich sowohl konventionelle, nicht-winkelstabile oder winkelstabile/verblockende Schraubensysteme eignen. Wenn winkelstabile/verblockende Schraubensysteme verwendet werden, haben sich monoaxiale Systeme, bei denen nur eine vorgegebene Schraubenrichtung möglich ist (z.B. LCP-System der Firma SYNTHES), oder polyaxiale Schraubensysteme, bei denen eine freie Richtungswahl der Schraube möglich ist (z.B. Polyax der Fa. DEPUY oder NCB-System der Fa. ZIMMER) als vorteilhaft erwiesen.

Die Fixation der Basisplatten der erfindungsgemässen Implantate lässt sich mit mono- oder bikortikalen Schrauben realisieren. Ein Vorteil der monokortikalen Schrauben besteht darin, dass der Prothesenschaft oder Zementmantel des Prothesenschafts nicht von der Schraube tangiert wird, und damit keine Gefahr der Prothesenschaft-Lockerung besteht. Der Vorteil der bikortikalen Schrauben liegt im besseren Halt. Sollte es der Operateur bevorzugen, oder sollte eine Fixation mit Schrauben nicht in Frage kommen, kann auch mittels Kabelseilen, Drähten oder Bändern fixiert werden.

Eine Fixation mittels resorbierbarer oder nicht-resorbierbarer Fäden ist ebenfalls möglich, und hat den Vorteil, dass metallischer Abrieb, der eine Prothesenlockerung induzieren könnte, vermieden wird.

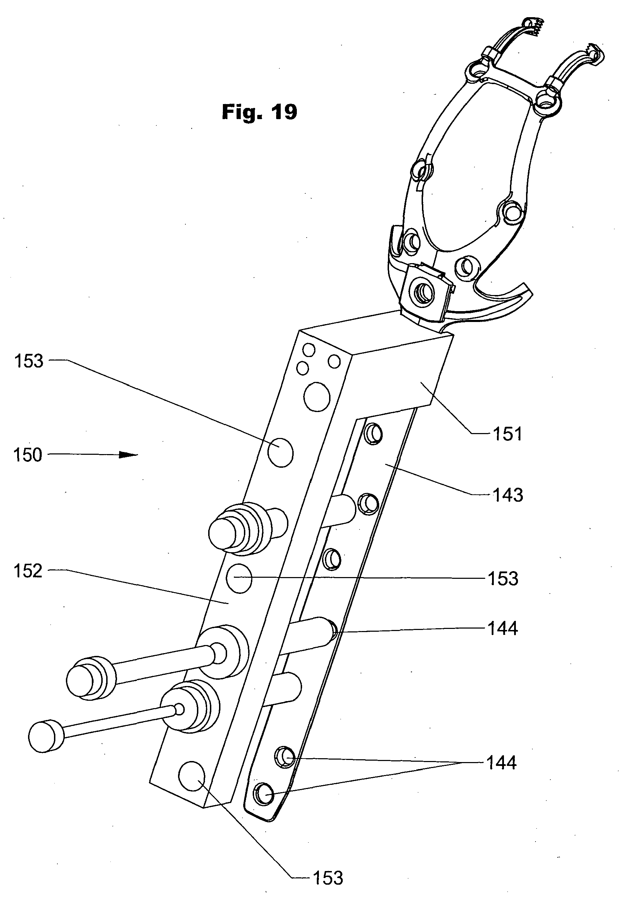

Für die minimal-invasive Implantation von Basisplatten mit langen Schäften, wie sie zum Beispiel in den Figuren 7 oder 14 bis 19 dargestellt sind, hat sich die Verwendung eines temporär an der Basisplatte montierten Zielbügels als vorteilhaft erwiesen, der die Besetzung der Plattenlöcher ermöglicht ohne den Muskel zu eröffnen und/oder abzuschieben. In der Figur 19 ist ein Zielbügel 150 gemäss einer bevorzugten Ausführungsform zusammen mit einer Basisplatte 143 gemäss einer weiteren Ausführungsform der Erfindung dargestellt. Der Zielbügel 150 ist in der Figur unterhalb der Klemmvorrichtung am proximalen Endbereich des Schaftes lösbar befestigt, so dass die Basisplatte 143 mit dem Zielbügel 150 temporär gehalten und unter den Musculuc vastus lateralis geschoben werden kann. Der L-förmige Zielbügel greift mit einem Abaxialstück 151, das im wesentlichen Senkrecht auf dem Schaft steht, am Schaft an. Ein rechtwinklig am Abaxialstück 151 angeordneter Bügelarm 152 verläuft positionsgenau parallel und beabstandet vom Schaft 143, so dass nach dem Anziehen der Kabelseile mittels Bohrhülsen 160 winkelstabile Schrauben durch den Bügelarm 152 des Zielbügels 150 in Schraubenlöcher 144 im Plattenschaft 143 eingebracht werden können. Korrespondierend zu den Schraubenlöchern im Plattenschaft 143 sind im Bügelarm 152 Positionierungsbohrungen 153 zur Aufnahme der Bohrhülsen 160 angeordnet. Im Ausführungsbeispiel der Figur 19 durchsetzen die Positionierungsbohrungen 153 den Bügelarm 152 im wesentlichen in senkrechter Richtung zum Schaft 143 hin, so dass die zu erstellenden Bohrungen und damit auch die Fixierschrauben senkrecht zur Femurlängsachse hin ausgerichtet sind. Ist eine andere Winkelstellung der Bohrungen und/oder der Schrauben im Knochen erwünscht, so kann die Lage und die Neigung der Positionierungsbohrungen im Bügelarm gegenüber dem Schaft entsprechend gewählt werden.

Gemäss einem bevorzugten minimal-invasiven Implantationsverfahren werden die Kabelseile in einem ersten Schritt, das heisst vor dem Einschieben der Basisplatte unter den Musculus vastus lateralis, vorverlegt. In einem zweiten Schritt wird die Basisplatte mit Hilfe eines Zielbügels eingeschoben und anschliessend werden die Kabelseile mit einem doppelseitigen Kabelspanner abgespannt und mittels der Klemmvorrichtung an der Basisplatte festgeklemmt. Der Zielbügel mit dem Bohrhülsen-Set wird anschliessend zum Erstellen von Bohrungen im Knochen und zum Eindrehen der winkelstabilen Schrauben verwendet und stellt sicher, dass die Fixierung exakt positioniert erfolgt, die Weichteiltraumatisierung aber auf einem minimalen Mass gehalten wird. Cerclagen mit Draht oder Kabelseil um Femur und Plattenschaft lassen sich bei montiertem Zielbügel nur schwierig anbringen.

Sollen nicht-winkelstabile Verschraubungen (d.h. Verschraubungen mit Schrauben ohne Gewinde im Kopfbereich) eingesetzt werden, so sind vorzugsweise Kombi-Löcher in der Basisplatte angebracht, wie sie zum Beispiel aus den LCP-Systemen der Firma Synthes bekannt sine. Diese Kombi-Löcher weisen zwei Bereiche auf, wobei in einem ersten Bereich eine Schraube mit Kompressionswirkung oder in einen zweiten Bereich des selben Loches eine winkelstabile Schraube eingebracht werden kann. Gleichzeitig können beide Schrauben nicht implantiert sein, wohl aber nacheinander. Wird ein Schaft mit Kombi-Löchern implantiert, so sind im Zielbügel entsprechend zwei Positionierungsbohrungen je Loch nötig, wobei die Positionierungsbohrung für das Anbringen der winkelstabilen Schraube entsprechend der gewünschten Winkelstellung geneigt verläuft.

Besonders bevorzugt werden die erfindungsgemässen Implantate, respektive deren Basisplatten durch eine Kombination der oben beschriebenen Techniken fixiert. So verhindern zum Beispiel monokortikale winkelstabile Schrauben (mono- oder polyaxial) einen "Scheibenwischereffekt" des Plattenschafts ohne den implantierten Schaft der Hüftgelenksprothese oder deren Zementmantel zu tangieren, und zusätzliche Cerclagen mit Kabelseilen verleihen zusätzliche Stabilität.

Grundsätzlich gilt, dass die Zinken und Flügel der erfindungsgemässen Basisplatten Herstellerseitig vorgebogen oder gerade sein können. Gerade Ausführungsformen können durch intraoperatives Biegen ganz nach Wunsch des Operateurs den Verhältnissen am Knochen angepasst werden. Vorzugswiese werden aber partiell oder vollständig vorgebogene Varianten eingesetzt, die nicht nur eine Zeitersparnis während der Operation bringen, sondern im Fall von vorgebogenen Zinken auch helfen das Trochanter-Fragment zu fassen und zu reponieren. Im Fall der Zinken haben sich partiell vorgebogene Varianten als besonders vorteilhaft erwiesen.

Die Zinken sind vorzugsweise biegsam und lassen durch Anziehen der Kabelseile ein perfektes Anpassen an die individuelle Anatomie der Trochanterspitze, respektive des TrochanterFragments zu. Ein sehr hohes Mass an Stabilität lässt sich durch einen geschlossenen Kabelseilverlauf erreichen, bei dem die über die Zinken geführten Kabelseile noch weiter nach lateral- distal bis hin zur Klemmvorrichtung geführt sind.

Wenn Eigenstabilität oder Hilfsmittel die Führung des biegsamen Gliedes sicherstellen, so genügt es ein solches Glied, zum Beispiel in Form eines Kabelseils medial anzuordnen. Entsprechend ist gemäss einer solchen Ausführungsform auch nur ein Zinken notwendig.

Als mögliche, aber operativ aufwändige und damit wenig bevorzugte Alternativen haben sich Implantate mit lateralem Zinkenverlauf und medialem Kabelverlauf erwiesen. Kabelseile, die fest mit der Basisplatte verbunden sind, z.B. an der medialen Spitze der Zinken angelötet oder angeklebt sind, sind hingegen eine praktikable Alternative zu den oben beschriebenen Kabelseilen zum Einschlaufen oder Einziehen.

Das mediale Verblocken der Zinken kann gemäss weiterer Ausführungsformen auch ohne ein Kreuzen der Kabelseile medial erreicht werden indem die Kabelseile medial durch eine Öse, einen Ring oder eine Klammer geschlauft werden, so dass auf das Überkreuzen verzichtet werden kann.

Bei einer weiteren Ausführungsform mit einem Kabelseil und zwei Zinken wird das eine Kabelseile durch Ösen an den Enden der Zinken oder Haken an denselben durchgeschlauft oder macht an diesen fest, so dass sich die Zinken beim Anziehen dieses Kabelseils wie ursprünglich vorgesehen biegen bis sie medial in Kontakt miteinander treten und beide Enden des Kabelseils im gespannten Zustand an der Klemmvorrichtung befestigt werden können.

Die Figur 15 zeigt in einer Detailvergrösserung ein zugespitztes Schaftende, das das Einschieben des Schaftes unter den M. vastus lateralis erleichtert und bei verschiedenen Ausführungsformen, insbesondere bei Implantaten zur minimal-invasiven Implantation realisiert werden kann.

### Liste der Bezugszeichen

- 1: Basisplatte
- 2: Schenkelknochen, Femur
- 3: unterer Abschnitt
- 4: oberer Abschnitt
- 5: flächenhafter Hauptteil
- 6: schmaler Schenkel
- 7: schmaler Schenkel
- 8: Trochantermassiv/proximaler Femur
- 9: Trochanter-Fragment/Trochanter major
- 10: Tuberculum innominatum
- 11: Grundkörper von 6
- 12: Grundkörper von 7
- 13: flächenhafter Steg
- 15: Fossa trochanterica
- 16: Zinken
- 17: Zinken
- 18: Zinkenbasis
- 19: Endpartie, verdickter Fortsatz
- 20: Haltevorrichtung
- 21: biegsames Glied von 41
- 22: biegsames Glied von 42
- 23: Bohrung durch 19
- 25: Kappe auf 46
- 26: länglicher Ausläufer
- 27: länglicher Ausläufer
- 28: Bohrung durch 29
- 29: freiliegende Endpartie von 26/27
- 30: Klammervorrichtung
- 31: Klemmplatte
- 32: Feingewindeschraube
- 33: Vertiefungen
- 36: Schrauben
- 37: Rand
- 38: unterer Rand von 5
- 39: oberer Rand von 5
- 41: Zügel
- 42: Zügel
- 43: Mittelteil der Zinkenbereiche
- 44: Stirnfläche von 19
- 45: zweite Mündung von 23
- 46: Endabschnitt von 141
- 48: zweite Mündung
- 49: Stirnfläche von 29
- 50: Fixiervorrichtungen bzw. Kabel-Cerclagen
- 53: Kabel/längliches und biegsames Glied
- 54: Klemmmittel
- 55: Seilende/Endpartie von 53
- 56: Seilende/Endpartie von 53
- 57: Grundkörper von 54
- 58: durchgehende Bohrung in 57
- 59: durchgehende Bohrung in 57
- 60: Halter/Ringführer
- 61: Konus/Grundkörper von 60
- 62: obere Stirnfläche von 61
- 63: untere Stirnfläche von 61
- 64: Mantel von 61
- 65: Öse
- 67: Nut
- 68: Langloch in 81
- 69: Langloch in 81
- 70: Ringhalter
- 71: Grundkörper von 70
- 72: Loch in 82
- 73: Loch in 82
- 74: Loch in 83
- 75: freie Endpartie von 76
- 76: Stirnfläche von 71
- 77: Nut
- 78: Langloch
- 79: Langloch
- 80: Zapfen
- 81: oberer Bereich von 3
- 82: mittlerer Bereich von 3
- 83: unterer Bereich von 3
- 100: Basisplatte
- 101: Plattenschaft schmal
- 102: Plattenschaft zweiflüglig
- 103: Längsflügel von 102
- 104: Längsflügel von 102
- 105: Plattenschaft breit
- 106: Schaftsegment
- 107: Schaftsegment
- 108: Schaftsegment
- 109: Basisplatte
- 110: Plattenschaft
- 111: Flügel
- 112: Flügel
- 113: Flügel
- 114: Basisplatte
- 115: Zinken
- 116: Zinken
- 120: Basisplatte
- 121: Zinken
- 122: Zinken
- 123: Quersteg
- 124: Verdickung
- 125: Verdickung
- 126: Führungsnut
- 127: Führungsnut
- 128: Verdickung
- 129: Verdickung
- 130: Klemmvorrichtung
- 131: Schaft
- 132: Klemmplatte mit Ausläufern
- 133: Ausläufer
- 134: Ausläufer
- 135: Durchführöffnung
- 136: Vertiefung/Nut
- 137: Innenseite
- 138: Innenseite
- 139: Schraubenloch
- 140: Abstandshalter
- 141: erste Endpartie von 41
- 142: zweite/freie Endpartie von 41
- 143: Plattenschaft
- 144: Schraubenloch
- 150: Zielbügel
- 151: Abaxialstück
- 152: Bügelarm
- 153: Positionierungsbohrung
- 160: Bohrhülse
- 351: Langloch in 4
- 352: Langloch in 4
- 353: Langloch in 4
- 354: Langloch in 4
- 355: Schraubenloch
- 356: Schraubenloch

## Patentansprüche

1. Trochanter-Zügelplatte für die Refixation des osteotomierten oder frakturierten Trochanter major (9) mit einer am proximalen Femur (2) fixierbaren Basisplatte (1, 100, 102, 105, 109, 114, 120), welche kraftschlüssig am Femur (2) gehalten wird und als Basis einer über den Trochanter major (9) verlaufenden Zuggurtungskonstruktion dient, die mindestens einen, vorzugsweise zwei, Zügel (41, 42) umfasst und den osteotomierten oder frakturierten Trochanter major form- und/oder kraftschlüssig am Femur (2) refixieren kann, **dadurch gekennzeichnet, dass** an der Basisplatte (1, 100, 102, 105, 109, 114, 120) im am Femur (2) fixierten Zustand proximal zwei Zinken (16, 17, 116, 117, 121, 122) angeordnet sind, wobei die Zinken (16, 17, 116, 117, 121, 122) mit daran angreifenden flexiblen, länglichen Gliedern (21, 22) die Zügel (41, 42) der geschlossenen Zuggurtungskonstruktion bilden.

2. Trochanter-Zügelplatte nach Patentanspruch 1, **dadurch gekennzeichnet, dass** die Basisplatte (1, 100, 102, 105, 109, 114, 120) vorzugsweise starr, die Zinken (16, 17, 116, 117, 121, 122) vorzugsweise biegsam und die flexiblen, länglichen Glieder (21, 22) vorzugsweise als Kabelseile oder Bänder ausgebildet sind.

3. Trochanter-Zügelplatte nach Patentanspruch 1 oder 2, **dadurch gekennzeichnet, dass** die Zinken (16, 17, 116, 117, 121, 122) beabstandet voneinander mit einer ersten Endpartie an der Basisplatte (1, 100, 102, 105, 109, 114, 120) angeschlossen sind und dass zumindest eine andere, freie Endpartie der jeweiligen Zinken (16, 17, 116, 117, 121, 122) mit einem biegsamen, länglichen Glied (21, 22) in Wirkverbindung steht.

4. Trochanter-Zügelplatte nach Patentanspruch 3, **dadurch gekennzeichnet, dass** die flexiblen, länglichen Glieder (21, 22) mit einer ersten Endpartie an der Basisplatte (1, 100, 102, 105, 109, 114, 120), im Bereich der ersten Endpartie oder im Bereich der anderen, freien Endpartie des jeweiligen Zinkens (16, 17, 116, 117, 121, 122) einerends angeschlossen sind und dass eine andere freie Endpartie (142) der flexiblen, länglichen Glieder (21, 22), zur Basisplatte (1, 100, 102, 105, 109, 114, 120) zurückläuft und dort fixiert ist.

5. Trochanter-Zügelplatte nach einem der Patentansprüche 1 bis 4, **dadurch gekennzeichnet, dass** mittels Zug an den flexiblen, länglichen Gliedern (21, 22) die freien Endpartien der Zinken (16, 17, 116, 117, 121, 122) verblockbar sind, was die intrinsische Stabilität der Zuggurtungskonstruktion erhöht.

6. Trochanter-Zügelplatte nach Patentanspruch 5, **dadurch gekennzeichnet, dass** die Basisplatte (1, 100, 102, 105, 109, 114, 120) lateral am proximalen Femur fixierbar ist, die freien Endpartien der Zinken (16, 17, 116, 117, 121, 122) medial des Trochanter major verblockbar sind und die flexiblen, länglichen Glieder (21, 22) zur Basisplatte (1, 100, 102, 105, 109, 114, 120) zurücklaufen, vorzugsweise nachdem sich diese mindestens über der Fossa trochanterica (15) und besonders bevorzugt nochmals unterhalb des Trochanter minor gekreuzt haben.

7. Trochanter-Zügelplatte nach einem der Patentanspruche 1 bis 6, **dadurch gekennzeichnet, dass** die Basisplatte (1) einen unteren, distalen Abschnitt (3) und einen oberen, proximalen Abschnitt (4) aufweist, der wiederum einen flächenhaften Hauptteil (5) und sich beabstandet voneinander daran angeordnete Schenkel (6, 7) umfasst, die sich im am Femur (2) fixierten Zustand nach proximal erstrecken, so dass die vorgenannten Anteile des proximalen Abschnitts (4) das Tuberculum innominatum umschließen, wobei die Schenkel (6, 7) vorzugsweise an ihrem proximalen Ende über einen Steg (13, 123) miteinander verbunden sind.

## Claims

1. A trochanter retention plate for refixation of the osteotomised or fractured trochanter major (9), having a base plate (1, 100, 102, 105, 109, 114, 120), which can be fixed to the proximal femur (2) and which is held in a force-fitting manner on the femur (2) and acts as a base for a tension band structure running over the trochanter major (9), comprising at least one, preferably two tension bands (41, 42) and being able to refix the osteotomised or fractured trochanter major on the femur (2) in a form-fitting and/or force-fitting manner, **characterised in that** two prongs (16, 17, 116, 117, 121, 122) are arranged proximally on the base plate (1, 100, 102, 105, 109, 114, 120) when fixed on the femur (2), wherein the prongs (16, 17, 116, 117, 121, 122) together with flexible, elongate members (21, 22) that act on said prongs form the tension bands (41, 42) of the closed tension band structure.

2. The trochanter retention plate according to claim 1, **characterised in that** the base plate (1, 100, 102, 105, 109, 114, 120) is preferably rigid, the prongs (16, 17, 116, 117, 121, 122) are preferably bendable, and the flexible, elongate members (21, 22) are preferably in the form of cables or bands.

3. The trochanter retention plate according to claim 1 or 2,**characterised in that** the prongs (16, 17, 116, 117, 121, 122) are attached to the base plate (1, 100, 102, 105, 109, 114, 120) at a distance from each other by means of a first end portion, and **in that** at least one other, free end portion of the respective prongs (16, 17, 116, 117, 121, 122) is operatively connected to a flexible, elongate member (21, 22).

4. The trochanter retention plate according to claim 3, **characterised in that** the flexible, elongate members (21, 22) are attached at one end to the base plate (1, 100, 102, 105, 109, 114, 120) by means of a first end portion, in the region of the first end portion or in the region of the other, free end portion of the respective prong (16, 17, 116, 117, 121, 122), and another free end portion (142) of the flexible, elongate members (21, 22) runs back to the base plate (1, 100, 102, 105, 109, 114, 120) and is fixed there.

5. The trochanter retention plate according to any one of claims 1 to 4, **characterised in that** the free end portions of the prongs (16, 17, 116, 117, 121, 122) can be locked by pulling on the flexible, elongate members (21, 22), which increases the intrinsic stability of the tension band structure.

6. The trochanter retention plate according to claim 5, **characterised in that** the base plate (1, 100, 102, 105, 109, 114, 120) can be fixed laterally to the proximal femur, the free end portions of the prongs (16, 17, 116, 117, 121, 122) can be locked medially of the trochanter major, and the flexible, elongate members (21, 22) run back to the base plate (1, 100, 102, 105, 109, 114, 120), preferably after they have intersected at least above the fossa trochanterica (15) and particularly preferably again below the trochanter minor.

7. The trochanter retention plate according to any one of claims 1 to 6, **characterised in that** the base plate (1) comprises a lower, distal section (3) and an upper, proximal section (4), which in turn comprises an extensive main part (5) and legs (6, 7), which are arranged thereon at a distance from each other and extend proximally when fixed to the femur (2), so that the above-mentioned parts of the proximal section (4) enclose the tuberculum innominatum, wherein the legs (6, 7) are preferably connected to each other at their proximal end by means of a web (13, 123).

## Revendications

1. Plaque de bridage du trochanter, pour la refixation du trochanter major (9) ayant subi une ostéotomie ou une fracture, avec une plaque de base (1, 100, 102, 105, 109, 114, 120) susceptible d'être fixée sur le fémur (2) proximal, que l'on maintient par complémentarité de force sur le fémur (2) et qui fait office de base pour une structure de membrure de traction s'étendant par-dessus le trochanter major (9) qui comprend au moins une, de préférence deux brides (41, 42) et est susceptible de refixer par complémentarité de forme et/ou par complémentarité de force le trochanter major ayant subi une ostéotomie ou une fracture sur le fémur (2), **caractérisée en ce que** sur la plaque de base (1, 100, 102, 105, 109, 114, 120), en position fixée sur le fémur (2), deux pointes (16, 17, 116, 117, 121, 122) sont disposées du côté proximal, dans lequel les pointes (16, 17, 116, 117, 121, 122) par des éléments (21, 22) allongés, flexibles s'engageant sur ces dernières, formant les brides (41, 42) de la structure de membrure de traction close.

2. Plaque de bridage du trochanter selon la revendication 1, **caractérisée en ce que** la plaque de base (1, 100, 102, 105, 109, 114, 120) est conçue de préférence en étant rigide, les pointes (16, 17, 116, 117, 121, 122) sont conçues de préférence en étant souples et les éléments (21, 22) allongés, flexibles sont conçus en tant que câbles ou en tant que bandes.

3. Plaque de bridage du trochanter selon la revendication 1 ou la revendication 2, **caractérisée en ce que** les pointes (16, 17, 116, 117, 121 ,122) sont raccordées en étant écartées l'une de l'autre par une première partie d'extrémité sur la plaque de base (1, 100, 102, 105, 109, 114, 120) et **en ce qu'**au moins une autre partie d'extrémité libre des pointes (16, 17, 116, 117, 121, 122) respectives est en liaison active avec un élément (21, 22) allongé flexible.

4. Plaque de bridage du trochanter selon la revendication 3, **caractérisée en ce que** les éléments (21, 22) allongés flexibles sont raccordées d'une part par une première partie d'extrémité sur la plaque de base (1, 100, 102, 105, 109, 114, 120), dans la région de la première partie d'extrémité ou dans la région de la deuxième partie d'extrémité libre de la pointe (16, 17, 116, 117, 121, 122) respective et **en ce qu'**une autre partie d'extrémité (142) libre des éléments (21, 22) allongés, flexibles retourne vers la plaque de base (1, 100, 102, 105, 109, 114, 120) et y est fixée.

5. Plaque de bridage du trochanter selon l'une quelconque des revendications 1 à 4, **caractérisée en ce que** par traction sur les éléments (21, 22) allongés flexibles, les parties d'extrémité libres des pointes (16, 17, 116, 117, 121, 122) sont susceptibles d'être bloquées, ce qui augmente la stabilité intrinsèque de la structure de membrure de traction.

6. Plaque de bridage du trochanter selon la revendication 5, **caractérisée en ce que** la plaque de base (1, 100, 102, 105, 109, 114, 120) est susceptible d'être fixée latéralement sur le fémur proximal, les parties d'extrémité libres des pointes (16, 17, 116, 117, 121, 122) sont susceptibles d'être bloquées sur le côté médial du trochanter major et les éléments (21, 22) allongés flexibles retournent vers la plaque de base (1, 100, 102, 105, 109, 114, 120), de préférence après s'être croisés au-dessus de la fossa trochanterica (15) et particulièrement de préférence encore une fois en dessous du trochanter minor.

7. Plaque de bridage du trochanter selon l'une quelconque des revendications 1 à 6, **caractérisée en ce que** la plaque de base (1) comporte une section (3) inférieure distale et une section (4) supérieure proximale, qui comprend quant à elle une pièce principale (5) plane et des branches (6, 7) placées sur cette dernière en étant écartées les unes des autres qui en position fixée sur le fémur (2) s'étendent en direction proximale, de sorte que les parties précédemment citées de la section (4) proximale entoure le tuberculum innominatum, les branches (6, 7) étant reliées entre elles, de préférence sur leur extrémité proximale par l'intermédiaire d'un listel (13, 123).
